# EUROPEAN PATENT SPECIFICATION

(11) **EP 1 796 483 B1**
(45) Date of publication and mention of the grant of the patent: **13.04.2016**
(21) Application number: 05741789.1
(22) Date of filing: 20.05.2005
(51) Int. Cl.: A23G 3/00

(54) **TOFFEE GUM COMPRISING CHOCOLATE**
SCHOKOLADEHALTIGER SAHNEKARAMELGUMMI
BONBON A MACHER CONTENANT DU CHOCOLAT

(30) Priority: 08.10.2004 WO PCT/DK2004/000691
(43) Date of publication of application: 20.06.2007
(73) Proprietor: Gumlink A/S, 7100 Vejle (DK)
(72) Inventor: TOPSØE, Martin, DK-7100 Vejle (DK); LUND, Kirsten, DK-7130 Juelsminde (DK)
(74) Representative: Patentgruppen
(86) International application number: PCT/DK2005/000335
(87) International publication number: WO 2006/037322

(56) References cited:
- EP-A- 0 372 695
- WO-A-96/20609
- US-A- 4 486 452
- US-A- 4 794 003
- US-A- 5 116 626
- US-A1- 2004 013 776

## Description

### Field OF THE INVENTION

The invention relates to a toffee gum comprising chocolate according to claim 1.

### BACKGROUND OF THE INVENTION

A group of conventional confectionery products include tablet-formed substances and/or chewable substances, which may be gradually dissolved or chewed and digested, such as candy, jelly, wine gum, liquorice, toffee, etc. This group of confectionery products generally benefits from a large variety of applicable textures, as indicated by the above-mentioned different products.

A problem related to this group of confectionery products, such as e.g. liquorice, toffee, etc, is that these products are consumed relatively fast, so that the consumer repeatedly needs to reload. This may lead to over-consuming, which may be undesirable due to a large intake of calories. In recent years, much attention has been paid to this problem. However, too large calorie-intake due to large consumption of confectionery products seems to be an increasing problem in some countries.

US 4,486,452 and US 2004/013776 disclose toffee confectioneries comprising chocolate.

WO 96/20609, US 5,116,626 and EP 0 372 695 disclose confectionery products comprising a polymer system comprising polyvinyl acetate.

### SUMMARY OF THE INVENTION

The present invention relates to toffee gum comprising a polymer system, and at least 1% by weight of chocolate,
said polymer system constituting at least 10% by weight of said toffee gum,
at least 70% by weight of said polymer system comprising polyvinyl acetate,
said polyvinyl acetate having a glass transition temperature (T_{g}) in the range of 0 to 60 °C, and said chocolate constituting at the most 90% by weight of said toffee gum.

In an embodiment of the invention, said glass transition temperature (T_{g}) of said polyvinyl acetate is in the range of 18 to 55 °C, preferably in the range of 25 to 40 °C.

In an embodiment of the invention, said toffee gum comprises at least one flavor and at least one sweetener.

According to the present invention, a new confectionery product has been obtained having texture properties emulating toffee but comprising a part, which is retained in the mouth during chewing. This confectionery product according to the invention is referred to as 'toffee gum'. The toffee gum according to the invention has toffee-like properties, but it is not completely swallowed during use. The toffee-like properties may be maintained for a relatively long time, e.g. 10 - 20 minutes, compared to chewing of a conventional toffee, which is usually swallowed during a shorter period of time.

Basically, the part of the toffee gum being retained in the mouth is substantially formed by the polymer system and parts of further ingredients being retained in the polymer system. Furthermore, according to the present invention, the polymer system, which comprises one or several polymers, is essential in providing the desired toffee-like properties for a prolonged time compared to conventional toffee, caramel, liquorice, wine gum, etc. According to the invention, it is required in obtaining these effects that the polymer system constitutes at least 10% by weight of the toffee gum product.

Thus, the prolonged texture compared to conventional toffee is obtained by way of the polymer system, as the polymers are not ingested during chewing but rather forming part of a toffee-emulating polymer structure, which may be chewed in the same way as a chewing gum. The polymer system may be compared in function to the gum base of a chewing gum, but with significant textural differences.

Surprisingly, it has been found, according to the invention, that polyvinyl acetate (PVAc) has exactly such properties essential for obtaining a polymer system providing the toffee gum product with toffee-like texture. Accordingly, it has been found that by adding polyvinyl acetate in significant amounts, it is possible to imitate the textural properties of toffee and obtain a toffee gum product having properties resembling or emulating toffee. The significant amount of polyvinyl acetate according to the invention is at least 70%, preferably more, by weight of the polymer system.

A further, very advantageous feature of the invention is that the toffee-like texture is combined with an improved robustness to e.g. fats. Thus, chocolate fillings or coatings may be applied within the scope of the invention. In other words, the nature of the polymers applied for the obtaining of a toffee gum is in particular useful as a robust polymer system when combined with chocolate. In contrast, e.g. chewable confectionery products based on smaller amounts of polyvinyl acetate and e.g. chewing gum comprising amounts of elastomers tend to disintegrate when combined with chocolate.

According to the invention, sweetener, flavor and chocolate are added to the toffee gum as taste providing components, which are substantially swallowed during use of the toffee gum product. Dependent on the affinity for the polymers, specific ingredients may be retained in the toffee gum for shorter or longer times during chewing of the product. Specific desired taste profiles may be obtained by adjusting types and amounts of flavors and sweeteners.

According to an embodiment of the invention, a part of a toffee may be substituted by a polymer system. Thus the toffee texture may be provided by the polymer system and as it is not consumed during use, the texture may be maintained beyond a usual period of time of chewing a toffee. A correspondingly prolonged taste may be obtained due to the fact that some of the taste-providing substances are incorporated into the polymer system, both by initial mixing during manufacturing and/or by mixing occurring during the chewing-process performed by a consumer. Further ways of regulating the length of the taste sensation include, incorporation of emulsifiers in the toffee gum and choosing the taste-providing substances in view of their affinity for the polymer system.

Furthermore, according to the invention, the application of the significant amount of polyvinyl acetate in the polymer system makes it possible to obtain an improved release of ingredients such as flavors, sweeteners, and active ingredients.

Herein, the polymer system is used as a term covering the polymers applied in the toffee gum confectionery product according to the invention. The polymer system may comprise one or more types of polymers. Other ingredients, such as fillers, softeners, etc., should not be regarded as being part of the polymer system.

According to a preferred embodiment of the invention, a toffee gum may be a suitable substitute for conventional toffee or caramel. Toffee gum as a substitute for toffee, caramel, and further soft candies such as liquorice has the advantage of being a low-calorie substitute, which moreover provides prolonged taste sensation.

In an embodiment of the invention, said polymer system constitutes at least 12% by weight of said toffee gum.

In an embodiment of the invention, said polymer system constitutes in the range of 15% to 95%, preferably 20% to 85%, more preferably 25% to 75%, and most preferably 30% to 65% by weight of said toffee gum.

The quantity of polymer system in a toffee gum according to the invention may be regulated in order to obtain a certain desired balance between substance retained in the mouth when consuming the product and substance being swallowed by the user.

The applied amount of polymer system furthermore affects the consistency of the product in combination with further ingredients such as softeners, sweeteners and flavors.

According to the most preferred embodiment of the invention, the amount of polymer system is regulated within the range of 30 to 65 weight percent of the toffee gum. If the percentage of polymer system gets too low, it may cause difficulty with the coherence and shape of the product. On the other hand, if the percentage of polymer system is too high, it may cause the product to be less tasty and more tiring to consume and chew.

In an embodiment of the invention, said polymer system comprises at least one substantially non-elastomeric polymer in an amount in the range of 70% to 100%, preferably 80% to 100% by weight of said polymer system.

According to an embodiment of the invention, it has been found that it is a key feature of the toffee gum that it comprises a polymer system of which the main part is composed of substantially non-elastomeric polymers.

Polyvinyl acetates of molecular weight in the range of 1000 to 100000 g/mol and a glass transition temperature in the range of 18 to 50 °C may show very desirable non-elastomeric properties and are according to the invention very important for obtaining the toffee-like consistency of the toffee gum.

In an embodiment of the invention, the polymer system is substantially formed by polyvinyl acetate(s) alone.

According to a preferred embodiment of the invention, the toffee gum is substantially free of natural and synthetic elastomers, which are typically applied within the art of manufacturing chewing gum.

The toffee texture of the polymer-based confectionery product according to the invention is obtained through a substantial amount of polyvinyl acetate and avoiding use of substantial amounts of high-molecular weight polymers, in particular elastomers.

Basically, a polymer system being comprised solely of polyvinyl acetate(s) is preferred in the sense that e.g. natural resins tend to counteract the - in this context - desired toffee-like properties.

According to a preferred embodiment of the invention, conventional elastomers should be avoided in order to avoid the typical chewing gum-like elastomeric properties, and according to a most preferred embodiment of the invention, the polymers of the product consist of polyvinyl acetate.

Evidently, insignificant amounts of other polymers may be acceptable within the scope of the invention without compromising the principles of the invention, namely that the polyvinyl acetates alone provide a toffee-like texture.

In an embodiment of the invention, said polyvinyl acetate has a molecular weight (Mw) in the range of 1000 to 250000 g/mol, preferably in the range of 2000 to 100000 g/mol, and most preferably in the range of 15000 to 60000 g/mol.

In an embodiment of the invention, the polymer system comprises at least one low-molecular weight polyvinyl acetate having a molecular weight (Mw) of about 2000 to 40000 g/mol, and
at least one high-molecular weight PVAc having a molecular weight (Mw) of 40001 to 200000 g/mol.

In an embodiment of the invention, the polymer system comprises at least one low-molecular weight (Mw) polyvinyl acetate having a molecular weight of about 9000 to 30000 g/mol, preferably about 13000 to 21000 g/mol.

In an embodiment of the invention, the toffee gum comprises at least one low-molecular weight polyvinyl acetate having a molecular weight (Mw) of about 2000 to 40000 g/mol in an amount of at least 70%, preferably at least 90% by weight of the polymer system.

In an embodiment of the invention, the toffee gum comprises at least one high-molecular weight polyvinyl acetate having a molecular weight (Mw) of 40000 to 100000 in an amount from about 0.5 to 10 % by weight of the polymer system.

In an embodiment of the invention, at least 70%, preferably at least 90%, and most preferably about 100% by weight of said polymer system is polyvinyl acetate having a glass transition temperature (T_{g}) in the range of 0 to 60 °C, preferably 10 to 50 °C, and most preferably 20 to 40°C.

The most preferred T_{g} of the final toffee gum may, according to an embodiment of the invention, lie within the range of 20 to 40 °C.

According to the invention, a toffee-like texture of the toffee gum confectionery product may be obtained when the polymer system is comprised mostly or completely of polyvinyl acetate having a glass transition temperature within the range of 20 to 40 °C.

According to an embodiment of the invention, the preferred polyvinyl acetate may be referred to as non-elastomeric and may also show resinous characteristics.

Such properties of the main part of the polymers are essential in order to obtain the desired consistency. Application of elastomers (elastomeric polymers) in too large amounts have been found to spoil the desired consistency - and make the product texture more chewing gum-like, which is far from the desired toffee-like consistency.

In an embodiment of the invention, at the most 30%, preferably at the most 10% by weight of said polymer system is polyvinyl acetate having molecular weight (Mw) in the range of 40001 - 200000 g/mol.

In an embodiment of the invention, said polymer system comprises less than 4% by weight of polymers having a molecular weight (Mw) from about 50000 to 99999 g/mol.

In an embodiment of the invention, said polymer system comprises less than 2% by weight of polymers having a molecular weight (Mw) from about 100000 to 199999 g/mol.

In an embodiment of the invention, said polymer system comprises less than 1% by weight of polymers having a molecular weight (Mw) from about 200000 to 499999 g/mol.

In an embodiment of the invention, said polymer system comprises less than 0.5% by weight of polymers having a molecular weight (Mw) from about 500000 to 10000000 g/mol.

According to an embodiment of the invention, high-molecular weight polymers, such as conventional elastomers or high-molecular weight polyvinyl acetate, should be kept low in concentration to obtain the desired texture.

Only a small amount of elastomeric polymers can be applied, without spoiling the desired texture of the toffee gum. They may be used for adjusting the texture, and may contribute with more robustness, but preferably elastomers are avoided.

It is crucial for obtaining the desired toffee texture that the amount of elastomeric polymer is kept low. Otherwise, the toffee gum is in danger of acquiring more chewing gum-like textural properties, which are undesired in the toffee gum of the invention.

In an embodiment of the invention, said polymer system is substantially free of elastomers.

According to an embodiment of the invention, the toffee gum confectionery product may be provided with the desired toffee-like texture on the basis of a polymer system, in which all the polymers comprised are substantially non-elastomeric polymers, mainly polyvinyl acetate, and none of them are elastomers.

In an embodiment of the invention, said toffee gum is characterised by having a storage modulus G' in the range of 100 Pa to 150000 Pa as measured at a frequency within the range of 0.1 to 10 Hz.

According to an embodiment of the invention, the storage modulus G" and the loss modulus G' are measured by AR 1000 rheometer from TA Instruments and at an oscillation torque of 10 µN·m and a temperature of 37°C.

In an embodiment of the invention, said toffee gum is characterised by having a loss modulus G" in the range of 1000 Pa to 200000 Pa as measured at a frequency within the range of 0.1 to 10 Hz.

In an embodiment of the invention, said toffee gum is characterised by having a ratio of said loss modulus G" to said storage modulus G' in the range of 1 to 5.

In an embodiment of the invention, said storage modulus G' is lower than said loss modulus G".

In an embodiment of the invention, said storage modulus G' is lower than said loss modulus G" as measured at a frequency of about 1 Hz.

In an embodiment of the invention, said loss modulus G" and said storage modulus G' are both lower than about 10000 Pa as measured at a frequency of about 0.1 Hz.

In an embodiment of the invention, said loss modulus G" is higher than about 10000 Pa as measured at a frequency of about 1 Hz.

In an embodiment of the invention, said storage modulus G' is lower than about 40000 Pa, preferably lower than about 30000 Pa as measured at a frequency of about 1 Hz.

In an embodiment of the invention, said storage modulus G' decreases during at least a part of a chewing process.

In an embodiment of the invention, said storage modulus G' decreases in the chewing process during the first 1 to 3 minutes.

In an embodiment of the invention, said loss modulus G" decreases during at least a part of the chewing process.

In an embodiment of the invention, said loss modulus G" decreases in the chewing process during the first 1 to 3 minutes.

In an embodiment of the invention, said chewing process involves a chewing frequency at about 1 Hz.

In an embodiment of the invention, said storage modulus G' and said loss modulus G" are measured at an oscillation torque of about 8 to 12 µN·m.

In an embodiment of the invention, said toffee gum comprises a toffee gum substance being formed from a mixture of said polymer system with at least one sweetener and at least one flavor.

In an embodiment of the invention, said toffee gum comprises toffee or caramel in an amount in the range of 1% to 85 % by weight of said toffee gum.

According to an embodiment of the invention, conventional toffee or caramel prepared from sugar or a sugar substitute such as isomalt or sorbitol may be added into the toffee gum as a supplement to the toffee gum substance being based on polyvinyl acetate.

In an embodiment of the invention, at least a part of said chocolate is forming or comprised in at least one chocolate module of said toffee gum.

According to different embodiments of the invention, the chocolate module may be composed of chocolate, only, or may be composed of a blend of chocolate, toffee, polymers and/or further ingredients.

In an embodiment of the invention, said at least one chocolate module comprises further ingredients.

The further ingredients in the chocolate module may e.g. be agents for modifying taste and consistency of the chocolate.

In an embodiment of the invention, at least one of said at least one chocolate module is forming an outer coating of said toffee gum.

The outer coating may comprise several layers, all of which or some of which comprise chocolate. A layer of chocolate may comprise further ingredients, e.g. for hardening or congealing of the chocolate.

In an embodiment of the invention, said toffee gum substance is forming a core and at least one of said at least one chocolate module is situated in said core

In an embodiment of the invention, said core comprises several of said at least one chocolate module.

In an embodiment of the invention, said chocolate is applied in an amount in the range of 5% to 80%, preferably 10% to 60% by weight of said toffee gum.

In an embodiment of the invention, said chocolate contains sugar.

In an embodiment of the invention, said chocolate is substantially sugar-free.

In an embodiment of the invention, said chocolate comprises cocoa butter in an amount of 15% to 90% by weight of said chocolate.

In an embodiment of the invention, said chocolate is selected from the group consisting of dark chocolate, milk, and white chocolate, or any combination thereof.

In an embodiment of the invention, said chocolate is selected from the group consisting of unsweetened chocolate, bittersweet chocolate, semisweet chocolate, and sweet chocolate, or any combination thereof.

In an embodiment of the invention, said chocolate is chocolate couverture.

In an embodiment of the invention, said chocolate is a compound chocolate.

In an embodiment of the invention, at least a part of said cocoa butter in said chocolate is replaced by an alternative vegetable fat or oil.

In an embodiment of the invention, said chocolate is substantially free of fat.

In an embodiment of the invention, said chocolate is selected from the group consisting of liquid chocolate, solid chocolate, and powdered chocolate, or any combination thereof.

In an embodiment of the invention, said chocolate module forming an outer coating has been applied by a coating method selected from the group consisting of spray coating, pan coating, curtain coating, or any combination thereof.

In an embodiment of the invention, said chocolate is substituted by a chocolate substitute.

In an embodiment of the invention, said toffee gum comprises a precoat below said outer coating. The precoat has been applied prior to the outer coating, which preferably comprises chocolate.

In an embodiment of the invention, said outer coating is supplied with a polishing. The polishing may preferably comprise gum arabic.

In an embodiment of the invention, said toffee gum is combined with further ingredients selected from the group consisting of coconut, rice, maize, nougat, marzipan, macaroon, almonds, fruits, berries, caramel, liquor, nuts, hazelnuts, peanuts, walnuts, liqourice, wine gum, or any combination thereof.

In an embodiment of the invention, said toffee gum comprises at least one softener in an amount in the range of 0.5% to 25%, preferably in the range of 1% to 18%, and most preferably in the range of 2% to 12% by weight of said toffee gum.

Softeners may be mixed with the polymers in order to soften the polymer system. The softened polymer system may be mixed with sweeteners, flavors, and etc., whereby a toffee gum according to the invention may be prepared.

As referred to herein, the term "softener" covers both softening compounds and compounds, which may be regarded as plasticizers.

In an embodiment of the invention, at least a part of said softener is incorporated in the polymer system, e.g. by mechanical mixing prior to the mixing of the polymer system with the further ingredients of the toffee gum. A mechanical mixing process may e.g. comprise extrusion or batch mixing processes such as those applied within the field of chewing gum.

According to an embodiment of the invention, softeners applied in a amount in the range of 0.5% to 25%, preferably in the range of 1% to 18%, and most preferably in the range of 2% to 12% by weight of the toffee gum, and combined with PVA, which comprises at least 70% PVAc by weight of polymer system, contributes significantly in obtaining the desired rheological properties, which are important for obtaining the desired toffee-like experience when consuming a toffee gum.

According to an embodiment of the invention, in which a chocolate coating is applied to the toffee gum, it may be preferred to apply the softener in the toffee gum substance in an amount of less than 18%, preferably less than 12%, and most preferably less than 8% by weight of the toffee gum, in order to maintain the robustness of the toffee gum towards the fat-containing chocolate.

In an embodiment of the invention, said at least one softener is selected from the group consisting of triacetin, lecithin, acetylated glycerides, mono- and di-glycerides, or any combination thereof.

According to an embodiment of the invention, the preferred softeners are mono- or di-glycerides. It has been found that these softeners are mild in the sense that they are not inclined to affect the texture of the toffee gum to an undesirable high extent. Thus, mono- or diglycerides are ideal as softeners for adjusting and fine-tuning of the toffee-like texture of the toffee gum confectionery product.

In an embodiment of the invention, said toffee gum comprises at least one syrup of a polyol in an amount of 1% to 25%, preferably 5% to 20% by weight of said toffee gum.

In an embodiment of the invention, the desired toffee-like texture may be obtained, when a considerable amount of polyol syrup is added to the toffee gum.

A quite large amount of polyol syrup in the final toffee gum may contribute to provide the toffee gum with very desirable textural properties according to the invention.

According to an embodiment of the invention, an advantageous syrup of a polyol, for application in the toffee gum, is maltitol syrup. According to an embodiment of the invention, maltitol syrup is a preferred choice as the polyol syrup for regulating the texture of the toffee gum and for softening the polyvinyl acetate.

According to an embodiment of the invention, a polyol syrup such as maltitol syrup may advantageously be applied in the toffee gum as a mildly softening compound, which is also conferring a nice sweet taste to the product.

According to an embodiment of the invention, application of a polyol syrup such as maltitol syrup in an amount in the range of 5 to 25 weight percent of the product may contribute significantly in obtaining the desired toffee-like consistency of the toffee gum.

In an embodiment of the invention, said polymer system comprises plasticizer(s).

According to an embodiment of the invention, plasticizers are applied for the purpose of obtaining a soft chew feel of the polyvinyl acetate based polymer system. It is noted that the desired plasticization depends heavily on the other ingredients applied in the product. As an example, some aggressive flavors, such as fruit flavors and acids, tend to soften the polymer system significantly compared to e.g. mint oil-based flavors.

Particularly useful plasticizers according to the present invention are triacetin, acetylated mono-and di-and triglycerides of short chain fatty acids, acetylated mono-and di-and triglycerides of medium-chain fatty acids, acetylated monoglycerides of long-chain fatty acids, methyl ester of rosin, low-molecular weight PVAc.

A preferred plasticizer will in a preferred embodiment have a hydrophilicity corresponding to the applied PVAc.

According to an embodiment of the invention, the polymer system comprises less than 30% by weight of plasticizers, preferably less than 10%, more preferably less than 8%, and most preferably less than 4% by weight of plasticizers.

According to a preferred embodiment of the invention, a relatively low amount of plasticizers should be applied in order to obtain the desired textural properties, i.e. the toffee-like chew feel. Moreover, off-notes may be present if too much plasticizer is present, especially when applying triacetin and glycerides. Finally, plasticizers such as triacetin and acetylated glycerides are expensive and the amount should be kept low.

In an embodiment of the invention, the toffee gum comprises less than 6%, preferably less than 4% by weight of filler, preferably less than 1% by weight.

In an embodiment of the invention, the toffee gum is substantially free of filler.

Thus, according to a preferred embodiment of the invention, fillers such as e.g. talc, should be avoided, as the desired toffee-like texture may be counteracted by the influencing of the filler on the mainly PVAc-based polymer system of the toffee gum.

In an embodiment of the invention, said toffee gum comprises at said least one flavor in an amount of about 0.001% to 30% by weight, and said at least one sweetener in an amount of about 5% to 80% by weight.

Flavors, sweeteners and further ingredients may be selected from those mentioned in the detailed description of the present invention.

In an embodiment of the invention, said flavor is a caramel flavor comprising an amount in the range of 1% to 30% by weight of said toffee gum substance.

In an embodiment of the invention, said at least one flavor is substantially oil-based and/or substantially hydrophilic flavors.

According to an embodiment of the invention, substantially oil-based flavors are preferred as such flavors tend to match the polymer system, which according to the invention may be regarded hydrophilic.

According to a further embodiment of the invention, substantially hydrophilic flavors have proved advantageous e.g. with respect to prolongation of release.

In an embodiment of the invention, said at least one sweetener comprises sugar.

In an embodiment of the invention, said sugar is selected from the group consisting of mono-, di-, or polysaccharides and any combination thereof.

In an embodiment of the invention, said sugar is selected from the group consisting of sucrose, dextrose, maltose, dextrins, trehalose, tagatose, dried invert sugar, fructose, levulose, galactose, corn syrup solids, polydextroseII, polydextrose ultra, inuline, or any combination thereof.

In an embodiment of the invention, said at least one sweetener is an artificial sweetener.

According to a preferred embodiment of the invention, artificial sweeteners may advantageously be applied in the toffee-gum-like confectionery product.

In an embodiment of the invention, said artificial sweetener is selected from the group consisting of sorbitol, mannitol, maltitol, xylitol, erythritol, lactitol, isomalt, derivatives of isomalt, or any combination thereof.

Accordingly, any other hydrogenated derivates of mono-, di-, or polysaccharides may be applied in the toffee gum of the invention. Furthermore, it is within the scope of the invention to apply sugar-sweeteners and artificial sweeteners in the same toffee gum composition.

In an embodiment of the invention, said artificial sweetener is a high-intensity artificial sweetener selected from the group consisting of aspartame, salts of acesulfame, alitame, neotame, twinsweet, saccharin and its salts, cyclamic acid and its salts, glycyrrhizin, dihydrochalcones, thaumatin, monellin, stevioside, sucralose, or any combination thereof.

In an embodiment of the invention, said toffee gum further comprises active ingredients.

According to an embodiment of the invention, applicable active ingredients may be selected among those listed in the detailed description.

In an embodiment of the invention, said toffee gum forms a tablet comprising a chewable polymer system.

In an embodiment of the invention, said toffee gum is formed in shapes such as cores, ellipsoid, balls, cylinders, squares, rectangular, hexagonal, strips, paraboloid, donut formed, ring formed, teddy bear formed and/or multi-modular.

In an embodiment of the invention, said toffee gum weighs from about ¼ gram to about 10 grams.

In an embodiment of the invention, said toffee gum weighs from about ½ gram to about 5 grams.

In an embodiment of the invention, said toffee gum is provided with an outer coating.

In an embodiment of the invention, said coating is selected from the group consisting of hard coating, soft coating and edible film-coating.

In an embodiment of the invention, said toffee gum is center-filled.

In an embodiment of the invention, said toffee gum is compressed.

According to an embodiment of the invention, it may be advantageous to apply compression techniques in the manufacture of the toffee gum. E.g. when incorporating ingredients being vulnerable to elevated temperatures or mixing processes, it may be preferable to apply compression in the preparation of the toffee gum of the present invention.

According to an embodiment of the invention, the compression of toffee gum refers to compression of the toffee gum substance or toffee gum core and excludes the chocolate module of the toffee gum.

The invention further relates to a method of manufacturing a toffee gum according to any of the claims 1 - 80, whereby the product is manufactured by a batch process.

A well-known batch manufacturing method is a two-step process according to which the polymer system (within the terms of chewing gum referred to as the gum base) is mixed in a first step on the basis of substantially hydrophobic components and where upon the further hydrophilic components such as sweeteners etc. are mixed together with the polymer system.

Furthermore, the invention relates to a method of manufacturing a toffee gum according to any of the claims 1 - 80, whereby the product is manufactured by an extruder process.

Manufacturing of the confectionery product and/or the polymer system of the product may advantageously be performed by extruding. The process is very attractive and advantageous in relation to the present invention due to the fact that the polymer system (the gum base equivalent) comprises very few components, e.g. preferably three components: a LMw PVAc, a HMw PVAc, and a plasticizer, e.g. triacetin.

The invention also relates to a method of coating a toffee gum according to any of the claims 1 - 80 by chocolate comprising the steps of applying chocolate in liquid form to the surface of a toffee gum core and subjecting the chocolate coated toffee gum to a temperature, which causes the liquid chocolate to harden.

### The figures

The invention will be described with reference to the figures where
- fig. 1A to 6: illustrate cross-sections of toffee gum pieces showing different examples of arrangements of toffee gum substance and chocolate in toffee gums according to the invention,
- fig. 7: illustrates cross-sections of small toffee gum pieces according to the invention, and
- fig. 8: illustrates frequency sweep measurements comparing toffee gum samples according to the invention with chewing gum samples.

### DETAILED DESCRIPTION

According to the present invention, a confectionery product has been provided, which has striking resemblance with toffee-products, but which is based on a polymer system mainly composed of polyvinyl acetate. The polymer system is mixed with further ingredients, at least comprising sweetener and flavor, and the mixture forms a confectionery substance, which is herein referred to as a toffee gum substance.

Surprisingly, the obtained toffee gum substance has considerable resistance towards fats and hence a great stability when combined with chocolate. Conversely, the resistance of the toffee gum substance towards moisture is limited, and it has actually been discovered that a coating of chocolate provides protection and prolonged shelf-life to the toffee gum substance.

Generally, according to the present invention, the provided toffee gum confectionery product typically comprises a polymer system and additional ingredients comprising flavoring agents, sweetening agents, texture-modifying agents, further optional ingredients and chocolate. Most part of the additional ingredients may typically be water-soluble. However, lipo-soluble ingredients may be fully applicable, such as for example flavoring oils. The chocolate may preferably form one or more modules. The chocolate modules are according to the invention situated in one or more regions in the toffee gum core or as a coating surrounding the toffee gum core.

The polymer system may include one or more polymers and may be mixed with a softening agent. The polymers are retained in the mouth and not swallowed during use, which has the advantageous effect of prolonging the toffee-like experience compared to conventional toffee products, which are normally completely swallowed.

Softening agents may be mixed into the polymers before addition of further ingredients, or mixed into the toffee gum composition at any time during the mixing procedure. In an embodiment of the invention, a part of the softening agents are added early in the process, e.g. added to the polymers alone, and further parts of the softening agents are added later on in the mixing process of the toffee gum.

Different kinds of softening agents may be useful for adjusting the texture of a specific polymer system. The exact composition of the polymer system and specific choice of additional ingredients, including softening, flavoring and sweetening agents, define the release properties of the toffee gum. The release rate of flavor and sweetener may be adjusted to be higher or lower during different phases of a chewing- and consuming-period of the toffee gum.

By addition of flavoring and sweetening agents into the toffee gum, any desired taste may be obtained, such as for example liquorice, chocolate, toffee, fruit, etc.

A way of characterizing the texture of the toffee gum is to measure the rheological properties, which involves the storage modulus G' and the loss modulus G" and the relation between the two at different frequencies. The term storage modulus G' may also be regarded as the elastic modulus, while the term loss modulus G" may be regarded as the viscous modulus. The ratio of G" to G', that is G"/G', is a measure of the relative importance of the viscous to elastic contributions for a material at a given frequency, and may be evaluated at a given oscillation torque and a given temperature.

The desired texture properties may be obtained by a proper combination of PVAc and softeners.

In the following, the toffee gum composition is described in further details, and examples are given of applicable components in the polymer system and of the additional ingredients in toffee gum products according to the invention.

In general, a toffee gum product composition according to the present invention typically comprises a water-soluble bulk portion, flavoring agents, and a water-insoluble chewable portion mainly composed of the polymer system. The water-soluble portion dissipates with a portion of the flavoring agent over a period of time during chewing, while the polymer system portion is retained in the mouth throughout the chew.

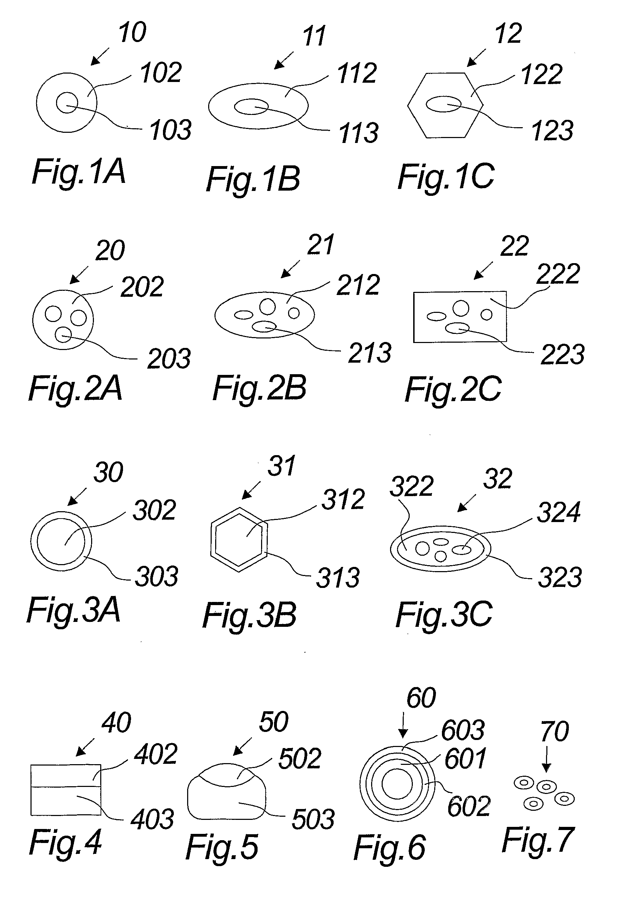

The addition of chocolate may be varied with regard to amount and arrangement in or around the toffee gum substance. See figures 1 to 7.

The polymer system provides the masticatory substance of the confectionery product according to the invention and greatly impart the chew characteristic s of the final toffee gum product. Furthermore, the polymer system affects the release profile of flavors, and sweeteners and plays a significant role in the final product with respect to both texture and taste. Some of the applied flavoring agents may have affinity for the polymer system and may be retained by the polymer system and hence be released over a prolonged time of chewing the toffee gum. Other flavoring agents may be readily released, and some may be released faster when emulsifiers are applied. Furthermore, emulsifiers may effect an improved stability towards humidity in the climate, as the emulsifiers may facilitate a lower uptake of water and thus a reduced tendency of the toffee gum to flow.

The water-insoluble portion of the toffee gum confectionery product contains the polymer system and typically a small amount of further ingredients such as plasticizers, waxes, softeners, fillers, colorants and antioxidants.

Preferably, the polymer system may comprise about 15% to about 95% by weight of the toffee gum confectionery product, more typically in the range of about 30% to about 65% by weight of the toffee gum.

The composition of the polymer system may vary depending on the particular product to be prepared and on the desired masticatory and other sensory characteristics of the final product. However, according to the invention, it has been found that polyvinyl acetate has properties, which are essential in obtaining a toffee-like texture of the confectionery product, and according to the invention polyvinyl acetate should always constitute the main part of the applied polymer system.

The polymer system may comprise polyvinyl acetate in an amount of 70% to 100% by weight of the polymer system. The polymer system may preferably be composed mostly of relatively low-molecular weight polyvinyl acetate, which may sometimes be referred to as resinous polymers. Elastomers and high-molecular weight polyvinyl acetate can only be applied to a very limited degree without compromising the desired toffee-like texture.

Softening agents may be incorporated into the polymer system to modify the texture. Softening agents may be added in an amount of 0% to 30% by weight in relation to the polymer system. Mild softening ingredients of the toffee gum such as polyol syrup, e.g. maltitol syrup, may within a most suitable range be added to the toffee gum in an amount of about 0% to 25% by weight of the final toffee gum substance.

The polymer system may further be mixed with fillers, waxes and fats. These components are generally not contributing to the desired to ffee-like texture properties, but fat in the form of a chocolate component is because of its taste a preferred ingredient in the toffee gum of the present invention. However, it should be noted that although it is possible to mix chocolate with the further toffee gum ingredients, chocolate is typically not mixed into the toffee gum composition like the rest of the ingredients in the toffee gum substance. Preferably, chocolate is situated in one or more modules in coherence with the toffee gum substance such as inner fillings or outer coatings.

Various arrangements of chocolate and toffee gum substance are applicable according to the invention. Some examples are illustrated in figures 1 to 7, wherein modules of chocolate and toffee gum substance are combined in different ways.

In fig. 1A, 1B, and 1C, toffee gums 10, 11, and 12 are illustrated in different shapes but all with one single inner filling of chocolate 103, 113, and 123. Toffee gum substances 102, 112, and 122 are surrounding the inner fillings of chocolate and forming the outer shape and surface of each toffee gum piece. Although not illustrated, a coating may be provided unto the toffee gum substance surface. Furthermore, the arrangement of toffee gum substance and chocolate may be reverse, whereby chocolate forms the outer material, and the inner filling is toffee gum substance.

In fig. 2A, 2B, and 2C, toffee gums 20, 21, and 22 are illustrated in different shapes and with several regions of inner filling of chocolate 203, 213, and 223. Toffee gum substances 202, 212, and 222 are surrounding the inner fillings of chocolate and forming the outer shape and surface of each toffee gum piece. Although not illustrated, a coating may be provided unto the toffee gum substance surface. Furthermore, the arrangement of toffee gum substance and chocolate may be reverse, whereby chocolate forms the outer material, and the inner fillings are formed from toffee gum substance.

In fig. 3A, 3B, and 3C, toffee gums 30, 31, and 32 are illustrated in different shapes and with outer coatings of chocolate 303, 313, and 323. Toffee gum substances 302, 312, and 322 are forming cores of toffee gum substance, which are surrounded and covered by the coatings of chocolate. Furthermore, in fig. 3C, the toffee gum 32 is provided with inner fillings of chocolate 324.

Fig. 4 illustrates a layered toffee gum piece 40, in which a layer of toffee gum substance 402 is combined with a layer of chocolate 403. The layers may be formed and combined by lamination or by compression. Although only two layers are shown in fig. 4, several layers of toffee gum substance and chocolate may be combined.

Fig. 5 illustrates a toffee gum piece 50 comprising a main part of chocolate 503 and a top part of toffee gum substance 502. The reverse may likewise be the case, whereby a main part of toffee gum substance may be provided with a top part of chocolate.

Fig. 6 illustrates a ring-formed toffee gum piece 60 formed of an inner ring 601, an intermediate ring 602, and an outer ring 603. The inner and outer rings may be formed from toffee gum substance, and the intermediate ring from chocolate, or reverse. Likewise, the toffee gum piece may be provided with further rings of chocolate or toffee gum substance. The intermediate ring 602, which may be made of chocolate, may be continuous through the toffee gum piece and visible on the surface of the toffee gum piece, or alternatively the chocolate intermediate ring 602 may be hidden inside a mass of toffee gum substance making it invisible without breaking the toffee gum piece.

Fig. 7 illustrates toffee gum pieces 70 as small pieces with a filling. The toffee gum pieces 70 may be formed of toffee gum substance with a filling or coating of chocolate. According to the invention, toffee gum may be provided as small pieces 70 intended for being consumed at once, instead of one single larger toffee gum piece.

Typically, the polymer system comprises 70% to 100% by weight of polyvinyl acetate having a glass transition temperature (T_{g}) in the range of 0 to 60 °C. The remainder 0% to 30% of the polymer system may be comprised of further synthetic resins, elastomers or high-molecular weight polyvinyl acetates. Preferably, the amount of elastomers does not exceed 10% by weight of the polymer system.

As referred to herein, the glass transition temperature (T_{g}) may be determined for example by DSC (DSC: differential scanning calorimetry). The DSC may generally be applied for determining and studying of the thermal transitions of a polymer and specifically, the technique may be applied for the determination of a second order transition of a material. The transition at T_{g} is regarded as such a second order transition, i.e. a thermal transition that involves a change in heat capacity, but does not have a latent heat. Hence, DSC may be applied for studying T_{g}.

According to an embodiment of the invention, the polyvinyl acetate, which comprises 70% to 100% by weight of the polymer system may have relatively low molecular weight (Mw). The low-molecular weight polyvinyl acetates, which according to an embodiment of the invention may be characterized as resinous, resin-like and non-elastomeric, may preferably have molecular weights (Mw) within the range of 1000 to 250000 g/mol, preferably 2000 to 200000 g/mol, and more preferably 4000 to 100000 g/mol. The polyvinyl acetate polymers may affect the firmness of the polymer system and toffee gum.

Unless otherwise indicated, as used herein with regard to polymers, the term "molecular weight" means weight average molecular weight (Mw) in g/mol.

Polyvinyl acetates of different molecular weights and the glass transition temperatures may be combined in the polymer system, and by their individual properties, the firmness, softness and further textural properties of the toffee gum may be varied.

High-molecular weight polyvinyl acetates of molecular weight (Mw) in the range of 25000 to 300000 g/mol, more commonly 30000 to 80000 g/mol may comprise up to 30%, preferably not more than 20%, and most preferably below 10% by weight of the polymer system. The higher the molecular weight, the less amount may be applied without spoiling the desired toffee-like properties of the toffee gum product according to the invention.

In accordance with the general principles in manufacturing a toffee gum product within the scope of the invention, variations of different suitable ingredients are listed and explained below.

Chocolate may be applied in an amount in the range of 1% to 90% by weight of the final toffee gum product according to the invention. The applicable chocolate types include dark chocolate, milk chocolate, white chocolate, chocolate couverture, unsweetened chocolate, bittersweet chocolate, semisweet chocolate, sweet chocolate, and compound chocolate. Both sugar-containing and sugar-free chocolate types may be applied. The compound chocolate refers to chocolate in which at least some of the cocoa butter is replaced by vegetable fat or oil substitutes. In general, any chocolate type and chocolate substitute known in the art may be applied according to the present invention. However, dependent on the desired taste and texture of the final product and dependent on the arrangement of the chocolate with respect to the toffee gum substance, such parameters as viscosity, hardening temperature, and taste of the chocolate should be taken into consideration. Specific examples of chocolate, which may be applicable, may e.g. be chocolate types such as those disclosed in EP0727146, US2003082291, WO00/02460, WO00/40101, WO01/56397, WO2005/016021, US4895732, US5279846, US6143345, hereby incorporated by reference, and in "The Materials Science of Chocolate" (Dec. 2000), by Peter Fryer and Kerstin Pinschower, MRS Bulletin. Further chocolate compositions for application in the present invention may for example be found in the books "Industrial Chocolate Manufacture and Use" (1999), Third edition, by S. T. Beckett, Blackwell Publishing Ltd, and "The Science of Chocolate" (2000), by S. T. Beckett, Royal Society of Chemistry, and "Chocolate, Cocoa & Confectionery" (1995), Third edition, by Bernard W. Minifie, Kluwer Academic Publishers, hereby incorporated by reference.

The chocolate taste and texture may e.g. be regulated by the degree of conching and by the contents of chocolate liquor, cocoa butter, sweetener, and e.g. vanilla, lecithin and milk solids. Likewise, the type and roasting of cocoa beans have effect on the chocolate quality. Different chocolate qualities may be applied as layers, coating, and filling of the toffee gum, respectively. For example, the viscosity of the chocolate should be relatively low, when applied as a layer, as e.g. showed in figure 4, and the chocolate viscosity may be high as well as low, when it is applied as an inner filling, as e.g. shown in figures 1 to 2. Correspondingly, the chocolate viscosity is important, when it is applied as a coating, as e.g. shown in figure 3. Here, a high viscosity, e.g. obtained by heating, is required during the coating procedure, and a hardening of the chocolate is required afterwards, e.g. obtained by cooling or by a dedicated chocolate quality.

Colorants, whiteners and antioxidants are optional ingredients, which may be mixed with the polymer system and may comprise an amount of up to about 5% by weight of the polymer system. The antioxidants may e.g. be chosen among butylated hydroxytoluene (BHT), butyl hydroxyanisol (BHA), propylgallate and tocopherols, and preservatives. The coloring agents and whiteners may for example comprise FD&C-type dyes and lakes, fruit or vegetable extracts, titanium dioxide, and combinations thereof.

In an embodiment of the invention, the toffee gum may comprise one or more softening agents in an amount of about 0 to about 18% by weight of the toffee gum, more typically about 0 to about 12% by weight of the toffee gum.

The softeners as well as emulsifiers may according to an embodiment of the invention be added both during mixing of the polymer system and later during mixing of the final toffee gum.

The softening ingredient may soften the polymer system and toffee gum formulation and may contribute to encompass ingredients such as waxes, fats, oils, emulsifiers, surfactants and solubilisers, which may optionally be added.

The softening agents e.g. sucrose esters may include those disclosed in WO 00/25598, which is incorporated herein by reference. Softening agents may further include tallow, hydrogenated tallow, hydrogenated and partially hydrogenated vegetable oils, cocoa butter, degreased cocoa powder, glycerol monostearate, glyceryl triacetate, lecithin, mono-, di- and triglycerides, acetylated monoglycerides, fatty acids (e.g. stearic, palmitic, oleic and linoleic acids) and combinations thereof. According to an embodiment of the invention, the preferred softener applied in the toffee gum may be triacetin.

Addition of one or more emulsifiers, which are also supplying softness to the product, may furthermore contribute to providing the product with water-binding properties and a pleasant smooth surface, and to reduce potential adhesive properties of the product. In an embodiment of the invention, the emulsifiers may comprise 0 to 18% by weight, preferably 0 to 12% by weight of the polymer system. Examples of emulsifiers may include mono- and diglycerides of edible fatty acids, lactic acid esters and acetic acid esters of mono- and diglycerides of edible fatty acids, acetylated mono and diglycerides, sugar esters of edible fatty acids, Na-, K-, Mg- and Ca-stearates, lecithin, hydroxylated lecithin and the like.

In case of the presence of biologically or pharmaceutically active ingredients as defined below, certain specific emulsifiers and/or solubilisers may be added in the toffee gum formulation in order to disperse and release these ingredients.

According to a preferred embodiment of the invention, the glass transition temperature(s) of the polymer system may be in the range of 0 to 50 °C, preferably within the range of 20 to 40 °C. The preferred T_{g} may be experienced by the polymer system alone or combined with one or more softeners, such as those mentioned above.

In an embodiment of the invention, waxes and fats may be used for adjustment of consistency and for softening of the toffee gum confectionery product. In connection with the present invention, examples of waxes and fats include for instance rice bran wax, polyethylene wax, petroleum wax (refined paraffin and microcrystalline wax), paraffin, beeswax, carnauba wax, candelilla wax, cocoa butter, degreased cocoa powder and any suitable oil or fat, as e.g. completely or partially hydrogenated vegetable oils or completely or partially hydrogenated animal fats.

Above-mentioned fats are not preferred in large amounts in the toffee gum of the present invention, as they have a tendency to counteract the desired toffee-like texture of the product. However, chocolate components such as cocoa butter and cocoa powder are preferred ingredients according to the present invention, although they are usually not mixed into the toffee gum composition in large amounts.

If waxes and/or fats are mixed into the toffee gum, the amount should be at the most 30% by weight in relation to the polymer system. Keeping the amount of fat low in the toffee gum composition may have the advantageous effect that the toffee gum may be more robust for an inner filling of fatty ingredients such as chocolate and for a soft coating, e.g. a coating with chocolate.

In an embodiment of the invention, the toffee gum may comprise filler in a small amount of about 0% to 10%, preferably 0% to 5% by weight of the toffee gum. However, within the range, the result of increasing the amount of filler may be that the final texture and consistence of the toffee gum may be tackier and lack more coherence. Examples of fillers and/or texturisers include magnesium and calcium carbonate, sodium sulphate, ground limestone, silicate compounds such as magnesium and aluminum silicate, kaolin and clay, aluminum oxide, silicium oxide, talc, titanium oxide, mono-, di- and tri-calcium phosphates, cellulose polymers, such as wood, and combinations thereof.

Typically, the most preferred texture according to the present invention may be obtained while avoiding fillers in the toffee gum composition.

Additional ingredients, which may be added to the toffee gum composition, are flavoring agents, bulk sweeteners, high-intensity sweeteners, acidulants, and other components that provide desired attributes.

Suitable bulk sweeteners include both sugar and non-sugar sweetening components. Bulk sweeteners typically constitute from about 5% to about 95% by weight of the toffee gum, more typically about 20% to about 80% by weight such as 30% to 60% by weight of the toffee gum.

Useful sugar sweeteners are saccharide-containing components including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High-intensity artificial sweetening agents can also be used alone or in combination with the above sweeteners. Preferred high-intensity sweeteners include, but are not limited to sucralose, aspartame, salts of acesulfame, alitame, neotame, twinsweet, saccharin and its salts, cyclamic acid and its salts, glycyrrhizin, dihydrochalcones, thaumatin, monellin, stevioside and the like, alone or in combination.

In order to provide longer lasting sweetness and flavor perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided using another component, which is already generally applied in the toffee gum such as a resinous compound.

Usage level of the high-intensity artificial sweetener will vary considerably and will depend on factors such as potency of the sweetener, rate of release, desired sweetness of the product, level and type of flavor used and cost considerations. Thus, the active level of high-potency artificial sweetener may vary from about 0% to ab out 8% by weight, preferably 0.001% to about 5% by weight. When carriers used for encapsulation are included, the usage level of the encapsulated sweetener will be proportionately higher.

Combinations of sugar and/or non-sugar sweeteners can be used in the toffee gum formulation processed in accordance with the invention. Additionally, the softener may also provide additional sweetness such as aqueous sugar or alditol solutions.

If a low-calorie product is desired, a low-caloric bulking agent may be used. Examples of low-caloric bulking agents include polydextrose, Raftilose, Raftilin, fructooligosaccharides (NutraFlora^{®}), palatinose oligosaccharides; guar gum hydrolysates (e.g. Sun Fiber^{®}) or indigestible dextrins (e.g. Fibersol^{®}). However, other low-caloric bulking agents may be used.

The toffee gum according to the present invention may contain aroma agents and flavoring agents including natural and synthetic flavorings e.g. in the form of natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint,' menthol, eucalyptus, clove oil, bay oil, anise, thyme, cedar leaf oil, nutmeg, and oils of the fruits mentioned above.

The toffee gum flavor may be a natural flavoring agent, which is freeze-dried, preferably in the form of a powder, slices or pieces or combinations thereof. The particle size may be less than 3 mm, less than 2 mm or more preferred less than 1 mm, calculated as the longest dimension of the particle. The natural flavoring agent may be in a form, where the particle size is from about 3 µm to 2 mm, such as from 4 µm to 1 mm. Preferred natural flavoring agents include seeds from fruit e.g. from strawberry, blackberry and raspberry.

Various synthetic flavors, such as mixed fruit flavors, may also be used in the composition of the toffee gum according to the invention. As indicated above, the aroma agent may be used in quantities smaller than those conventionally used. The aroma agents and/or flavors may be used in the amount from 0.01% to about 30% by weight of the final product depending on the desired intensity of the aroma and/or flavor used. Preferably, the content of aroma/flavor is in the range of 0.2% to 3% by weight of the total composition.

In an embodiment of the invention, the flavoring agents comprise natural and synthetic flavorings in the form of natural vegetable components, essential oils, essences, extracts, powders, including acids and other substances capable of affecting the taste profile.

Further toffee gum ingredients, which may be included in the toffee gum according to the present invention, include surfactants and/or solubilisers, especially when pharmaceutically or biologically active ingredients are present. As examples of types of surfactants to be used as solubilisers in a toffee gum composition according to the invention, reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or nonionic solubilisers can be used. Suitable solubilisers include lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllatylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. In the presence of an active ingredient, the toffee gum may preferably also comprise a carrier known in the arts of chewing gum and pharmaceutical ingredients.

Emulsifiers, which are used as softeners may include tallow, hydrogenated tallow, hydrogenated and partially hydrogenated vegetable oils, cocoa butter, glycerol monostearate, glycerol triacetate, lechithin, mono-, di-and triglycerides, acetylated monoglycerides, fatty acids (e.g. stearic, palmitic, oleic and linoleic acids), and combinations thereof.

According to an embodiment of the invention, the toffee gum may comprise a pharmaceutically, cosmetically or biologically active substance. Examples of such active substances, a comprehensive list of which is found e.g. in WO 00/25598, which is incorporated herein by reference,

The active agents to be used in connection with the present invention may be any substance desired to be released from the toffee gum. If an accelerated rate of release is desired, corresponding to the effect obtained for the flavor, the primary substances are those with limited water solubility, typically below 10g /100 ml including substances which are entirely water insoluble. Examples are medicines, dietary supplements, oral compositions, anti-smoking agents, highly potent sweeteners, pH adjusting agents, etc.

Further examples of active ingredients include paracetamol, benzocaine, cinnarizine, menthol, carvone, caffeine, chlorhexidine-di-acetate, cyclizine hydrochloride, 1,8-cineol, nandrolone, miconazole, mystatine, aspartame, sodium fluoride, nicotine, saccharin, cetylpyridinium chloride, other quaternary ammonium compounds, vitamin E, vitamin A, vitamin D, glibenclamide or derivatives thereof, progesterone, acetylsalicylic acid, dimenhydrinate, cyclizine, metronidazole, sodium hydrogen carbonate, the active components from ginkgo, the active components from propolis, the active components from ginseng, methadone, oil of peppermint, salicylamide, hydrocortisone or astemizole.

Examples of active agents in the form of dietary supplements are for instance salts and compounds having the nutritive effect of vitamin B2 (riboflavin), B12, folinic acid, niacine, biotine, poorly soluble glycerophosphates, amino acids, the vitamins A, D, E and K, minerals in the form of salts, complexes and compounds containing calcium, phosphorus, magnesium, iron, zinc, copper, iodine, manganese, chromium, selenium, molybdenum, potassium, sodium or cobalt.

Furthermore, reference is made to lists of nutritients accepted by the authorities in different countries such as for instance US code of Federal Regulations, Title 21, Section 182.5013.182 5997 and 182.8013-182.8997.

Examples of active agents in the form of compounds for the care or treatment of the oral cavity and the teeth, are for instance bound hydrogen peroxide and compounds capable of releasing urea during chewing.

Examples of active agents in the form of antiseptics are for instance salts and compounds of guanidine and biguanidine (for instance chlorhexidine diacetate) and the following types of substances with limited water-solubility: quaternary ammonium compounds (for instance ceramine, chloroxylenol, crystal violet, chloramine), aldehydes (for instance paraformaldehyde), compounds of dequaline, polynoxyline, phenols (for instance thymol, para chlorophenol, cresol) hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. furthermore Martindale, The Extra Pharmacopoeia, 28th edition, pages 547-578; metal salts, complexes and compounds with limited water-solubility, such as aluminum salts, (for instance aluminum potassium sulfate AIK (S04) 2, 12H20) and furthermore salts, complexes and compounds of boron, barium, strontium, iron, calcium, zinc, (zinc acetate, zinc chloride, zinc gluconate), copper (copper chloride, copper sulfate), lead, silver, magnesium, sodium, potassium, lithium, molybdenum, vanadium should be included; other compositions for the care of mouth and teeth: for instance; salts, complexes and compounds containing fluorine (such as sodium fluoride, sodiummonofluorophosphate, aminofluorides, stannous fluoride), phosphates, carbonates and selenium.

Cf. furthermore J. Dent. Res. Vol. 28 No. 2, page 160-171,1949, wherein a wide range of tested compounds are mentioned.

Examples of active agents in the form of agents adjusting the pH in the oral cavity include for instance: acceptable acids, such as adipinic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulfates or oxides of sodium, potassium, ammonium, magnesium or calcium, especially magnesium and calcium.

Examples of active agents in the form of anti-smoking agents include for instance: nicotine, tobacco powder or silver salts, for instance silver acetate, silver carbonate and silver nitrate.

Further examples of active agents are medicines of any type.

Examples of active agents in the form of medicines include caffeine, salicylic acid, salicylic amide and related substances (acetylsalicylic acid, choline salicylate, magnesium salicylate, sodium salicylate), paracetamol, salts of pentazocine (pentazocine hydrochloride and pentazocinelactate), buprenorphine hydrochloride, codeine hydrochloride and codeine phosphate, morphine and morphine salts (hydrochloride, sulfate, tartrate), methadone hydrochloride, ketobemidone and salts of ketobemidone (hydrochloride), beta-blockers, (propranolol), calcium antagonists, verapamil hydrochloride, nifedinpine as well as suitable substances and salts thereof mentioned in Pharm. Int., Nov. 85, pages 267-271, Barney H. Hunter and Robert L. Talbert, nitroglycerine, erythrityl tetranitrate, strychnine and salts thereof, lidocaine, tetracaine hydrochloride, etorphine hydrochloride, atropine, insulin, enzymes (for instance papain, trypsin, amyloglucosidase. glucoseoxidase, streptokinase, streptodornase, dextranase, alpha amylase), polypeptides (oxytocin, gonadorelin, (LH. RH), desmopressin acetate (DDAVP), isoxsuprine hydrochloride, ergotamine compounds, chloroquine (phosphate, sulfate), isosorbide, demoxytocin, heparin.

Other active ingredients include beta-lupeol, Letigen, Sildenafil citrate and derivatives thereof.

Dental products include Carbami, CPP Caseine Phospho Peptide; Chlorhexidine, Chlorhexidine di acetate, Chlorhexidine Chloride, Chlorhexidine di gluconate, Hexetedine, Strontium chloride, Potassium Chloride, Sodium bicarbonate, Sodium carbonate, Fluor containing ingredients, Fluorides, Sodium fluoride, Aluminum fluoride, Ammonium fluoride, Calcium fluoride, Stannous fluoride, Other fluor containing ingredients Ammonium fluorosilicate, Potasium fluorosilicate, Sodium fluorosilicate, Ammonium monofluorphosphate, Calcium monofluorphosphate, Potassium monofluorphosphate, Sodium monofluorphosphate, Octadecentyl Ammonium fluoride, Stearyl Trihydroxyethyl Propylenediamine Dihydrofluoride, Vitamins include A, B1, B2, B6, B12, Folinic acid, niacin, Pantothenacid, biotine, C, D, E, K.

Minerals include Calcium, phosphor, magnesium, iron, Zink, Cupper, Led, Mangan, Chromium, Selene and Molybden. Other active ingredients include: Q10@, enzymes. Natural drugs including Ginkgo Biloba, ginger, and fish oil. The invention also relates to use of migraine drugs such as Serotonin antagonists: Sumatriptan, Zolmitriptan, Naratriptan, Rizatriptan, Eletriptan; nausea drugs such as Cyclizin, Cinnarizin,
Dimenhydramin, Difenhydrinat; hay fever drugs such as Cetrizin, Loratidin, pain relief drugs such as Buprenorfin, Tramadol, oral disease drugs such as Miconazol, Amphotericin B, Triamcinolonaceton; and the drugs Cisaprid, Domperidon, Metoclopramid.

Active ingredients may comprise the below-mentioned compounds or derivates thereof but are not limited thereto: Acetaminophen, Acetyl salicylic acid Buprenorphine Bromhexin Celcoxib Codeine, Diphenhydramin, Diclofenac, Etoricoxib, Ibuprofen, Indometacin, Ketoprofen, Lumiracoxib, Morphine, Naproxen, Oxycodon, Parecoxib, Piroxicam, Pseudoefedrin, Rofecoxib, Tenoxicam, Tramadol, Valdecoxib, Calciumcarbonat, Magaldrate, Disulfiram, Bupropion, Nicotine, Azithromycin, Clarithromycin, Clotrimazole, Erythromycin, Tetracycline, Granisetron, Ondansetron, Prometazin, Tropisetron, Brompheniramine, Ceterizin, leco-Ceterizin, Chlorcyclizine, Chlorpheniramin, Chlorpheniramin, Difenhydramine, Doxylamine, Fenofenadin, Guaifenesin, Loratidin, des-Loratidin, Phenyltoloxamine, Promethazin, Pyridamine, Terfenadin, Troxerutin, Methyldopa, Methylphenidate, Benzalcon. Chloride, Benzeth. Chloride, Cetylpyrid. Chloride, Chlorhexidine, Ecabet-sodium, Haloperidol, Allopurinol, Colchinine, Theophylline, Propanolol, Prednisolone, Prednisone, Fluoride, Urea, Miconazole, Actot, Glibenclamide, Glipizide, Metformin, Miglitol, Repaglinide, Rosiglitazone, Apomorfin, Cialis, Sildenafil, Vardenafil, Diphenoxylate, Simethicone, Cimetidine, Famotidine, Ranitidine, Ratinidine, cetrizin, Loratadine, Aspirin, Benzocaine, Dextrometorphan, Ephedrine, Phenylpropanolamine, Pseudoephedrine, Cisapride, Domperidone, Metoclopramide, Acyclovir, Dioctylsulfosucc., Phenolphtalein, Almotriptan, Eletriptan, Ergotamine, Migea, Naratriptan, Rizatriptan, Sumatriptan, Zolmitriptan, Aluminum salts, Calcium salts, Ferro salts, Silver salts, Zinc-salts, Amphotericin B, Chlorhexidine, Miconazole, Triamcinolonacetonid, Melatonine, Phenobarbitol, Caffeine, Benzodiazepiner, Hydroxyzine, Meprobamate, Phenothiazine, Buclizine, Brometazine, Cinnarizine, Cyclizine, Difenhydramine, Dimenhydrinate, Buflomedil, Amphetamine, Caffeine, Ephedrine, Orlistat, Phenylephedrine, Phenylpropanolamin, Pseudoephedrine, Sibutramin, Ketoconazole, Nitroglycerin, Nystatin, Progesterone, Testosterone, Vitamin B12, Vitamin C, Vitamin A, Vitamin D, Vitamin E, Pilocarpin, Aluminumaminoacetat, Cimetidine, Esomeprazole, Famotidine, Lansoprazole, Magnesiumoxide, Nizatide and or Ratinidine.

In one embodiment of the invention, the flavor may be used as taste masking in a toffee gum comprising active ingredients, which themselves have undesired taste or which alter the taste of the formulation.

The toffee gum may optionally contain additives, such as binding agents, acidulants, fillers, coloring agents, preservatives, and antioxidants, for instance butylated hydroxytoluene (BHT), butyl hydroxyanisol (BHA), propylgallate and tocopherols.

Colorants and whiteners may include FD & C-type dyes and lakes, fruit and vegetable extracts, titanium dioxide, and combinations thereof.

Materials to be used for the above-mentioned encapsulation methods for sweeteners might e.g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Generally, it is preferred that the polymer system of the toffee gum products prepared according to the invention is based mainly or solely on polyvinyl acetate.

However, within the scope of the invention, small amounts, never exceeding 30% by weight of the polymer system, of further natural or synthetic resins, and synthetic elastomers may be applied. Examples are mentioned below.

Examples of synthetic resins, which should be limited in amount, include, vinyl acetate-vinyl laurate copolymers and terpene resins derived from alpha-pinene, betapinene, and/or d-limonene, and natural terpene resins. Examples of synthetic elastomers include those listed in Food and Drug Administration, CFR, Title 21, Section 172,615, the Masticatory Substances, Synthetic such as polyisobutylene. e.g. having a gel permeation chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyisoprene, polyethylene, vinyl acetate-vinyl laurate copolymer e.g. having a vinyl laurate content of about 5% to 50% by weight such as 10% to 45% by weight of the copolymer, and combinations hereof.

High- and low- molecular weight synthetic elastomers may be combined in the same polymer system. Examples of such combinations are polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer.

Examples of natural resins are: Natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins, pentaerythritol esters of rosins.

Although it is not preferred, it is within the scope of the invention to apply PVAc, which comprises a limited amount of water, such as in the range of 0.1 to 4% by weight of the PVAc.

Polymer systems of the invention are typically prepared by adding an amount of polyvinyl acetate, mostly low-molecular weight PVAc, and plasticizer to a heated (10°C - 120°C) sigma blade mixer with a front to rear speed ratio from about 1.2:1 to about 2:1, the higher ratio typically being used for a polymer system which requires more rigorous compounding of its medium/high-molecular weight polymers.

The initial amounts of ingredients comprising the initial mass may be determined by the working capacity of the mixing kettle in order to attain a proper consistency and by the degree of compounding desired to break down the although slight amount of medium/high-molecular weight polymers and increase chain branching. The longer the time of compounding, the use of lower molecular weight or softening point polymer system ingredients, the lower the viscosity and firmness of the final polymer system.

Compounding typically begins to be effective once the ingredients have massed together. Anywhere from 15 to 90 minutes may be the length of compounding time.

Preferably, the time of compounding is from 20 to about 60 minutes.

After the initial ingredients have massed homogeneously and compounded for the time desired, the balance of the polymer system ingredients are added in a sequential manner until a completely homogeneous molten mass is attained. Typically, any remainder of the polymer system components is added within 60 minutes after the initial compounding time.

Typical polymer system processing times may vary from about 0.5 to about 4 hours, preferably from about 0.5 to 1.5 hours, depending on the formulation. The final mass temperature when dumped may be between 70°C and 130°C and preferably between 100°C and 120°C. The completed molten mass is emptied from the mixing kettle into coated or lined pans, extruded or cast into any desirable shape and allowed to cool and solidify. Those skilled in the art will recognize that many variations of the above-described procedure may be followed.

In general, a toffee gum product according to the invention may be manufactured by sequentially adding the various ingredients to a commercially available mixer. After the ingredients have been thoroughly mixed, the toffee gum mass is discharged from the mixer and shaped into the desired form such as by rolling into sheets and cutting into sticks, extruded into chunks or casting into pellets.

A typical example of the mixing procedure may in an embodiment of the invention involve the following. Generally, the ingredients may be mixed by first softening and mixing the polymer system. The softening agents or part of them may also be added at this time. Colors, active agents and/or emulsifiers may then be added, along with syrup and a portion of the bulking agent/sweetener. Further portions of the bulking agent/sweetener may then be added into the mixer. A flavoring agent may typically be added with the final portion of the bulking agent/sweetener. A high-intensity sweetener may preferably be added after the final portion of bulking agent.

The entire mixing procedure typically takes from 12 to 25 minutes, but longer mixing times may sometimes be required. Many variations of the above-described procedure may be followed, including a one-step method such as described in US patent application 2004/0115305, hereby incorporated as reference. Toffee gum products may be formed by extrusion, compression, rolling and may be center-filled with liquids and/or solids in any form.

According to a preferred embodiment of the invention, the toffee gum is manufactured by an extrusion process.

Chocolate may be added as inner filling in the product through a continuous extruding process. Examples of conventional processes for manufacturing a confectionery product with an inner filling may be found in US4450179, US4468185, US5002791, US5035905, US5125819, US6759079, US6805890, US6838098, US2002/0192330, EP1206194, GB2018668, WO02/15708, WO02/19835, WO03105594, PCT/DK2005/000066, hereby incorporated by reference. Furthermore, chocolate may be added as a coating of the toffee gum, and conventional chocolate coating methods may be applied.

The toffee gum according to the present invention may also be provided with an outer coating, which may be a hard coating, a soft coating, a film coating, or a coating of any type that is known in the art, or a combination of such coatings. The coating may typically constitute 0.1 to 75 percent by weight of a coated toffee gum product.

According to a preferred embodiment of the invention, the toffee gum may be coated with chocolate, which may provide some advantages compared to film coating or coating by sugars or sugar substitutes. It has been found according to the invention, that chocolate coatings may provide a protection of the toffee gum core against the surroundings, and specifically humidity. The influence of humidity on toffee gum according to the invention may be considerable and may tend to damage the toffee gum texture and shape, but it has been found that a chocolate coating provides a considerable protection of the toffee gum core against humidity.

The toffee gum may be coated with chocolate following conventional chocolate coating processes, such as batch and continuous processes including e.g. coating in a pan or drum, spray coating, curtain coating, and coating by a flow of chocolate onto the toffee cores. The chocolate coating may comprise dark chocolate, white chocolate, milk chocolate, or any combination thereof. The coating may comprise several layers and have any thickness. Different chocolate types may be applied to provide different tastes and coating patterns, such as e.g. stripes, check, circles, etc.

In a continuous coating process according to an embodiment of the invention, toffee gum cores or articles, including toffee gum substance in any shape, are typically transferred to a conveyer belt and exposed to a spray, flow, or curtain of liquid chocolate, to coat the whole toffee gum core or just the top part. Excess chocolate, still in liquid form, may by itself flow off the conveyer belt to the sides or through holes of spacings in the conveyer belt construction. For example, the conveyer belt may have a rod-like construction, and thus have spacing between the rods providing passage for liquid chocolate. Soon after application of chocolate, while the chocolate is still at least partly liquid, the coated toffee gum articles may be subjected to a blow of air, e.g. from a nozzle, to remove more of the excess chocolate or to provide the chocolate coated toffee gum surface with a certain appearance. Excess chocolate may be collected and recycled in the coating process.

According to an embodiment of the invention, the bottom part of the toffee gum articles may in a continuous process be coated before, at the same time, or after coating of the top part. Coating of the bottom part may for example be carried out by use of an endless conveyer belt, which is continuously covered by liquid chocolate, so that chocolate is applied to the bottom part of the toffee gum articles being placed on the belt. As a further example, the bottom part of the toffee gum articles may be coated by a flow of chocolate, which reaches the toffee gum article s from below the conveyer belt and through passages, such as spacings, in the conveyer belt. The flow may e.g. be supplied by nozzles and/or piping below the conveyer belt.

In a batch coating process according to an embodiment of the invention, the toffee gum cores may be subjected to a number of application cycles, whereby a number of chocolate layers, typically about 3 to 30 layers, are applied to the toffee gum surface. The layers may be of the same type of chocolate or alternating types of chocolate, so as to form a certain desired coating appearance and thickness. Furthermore, the viscosity of the applied chocolate and the time for each coating cycle may be controlled in order to obtain the desired appearance and thickness of the final coating. The coating process may typically correspond to a so-called panning carried out in a drum-like coating pan by conventional chocolate coating processes.

According to an embodiment of the invention, the temperature during the chocolate coating process should be carefully controlled dependent on the type of chocolate applied. Typically, in some embodiments of the invention, the surrounding temperature during coating of the toffee gum articles is low enough for the chocolate to harden and/or solidify. The temperature may for example be controlled by supply of cold air. However, further cooling is sometimes needed subsequent to the coating process. Furthermore, both temperature and pressure may be adjusted in order to keep the chocolate in liquid form, while applying it to the surface of the toffee gum articles.

Subsequent to, or in continuation of, a coating process, the coated toffee gum articles may according to an embodiment of the invention be polished and/or sealed. Suitable mixtures for polishing are known within the art of coating, and likewise suitable materials for sealing are known, such as e.g. shellac.

Description of different applicable chocolate coating processes, which may be applied for coating of a toffee gum according to the invention, may for example be found in US6730344, US4486452, US4357359, WO03/059079, US4032667, US2915024, US5480664, US3470831, and US6251454, hereby incorporated by reference.

A further applicable outer coating type is a hard coating, which term is including sugar coatings and sugar-free (or sugarless) coatings and combinations thereof. The object of hard coating is to obtain a sweet, crunchy layer, which is appreciated by the consumer and to protect the center of the product. In a typical process of providing the toffee gum with a protective sugar coating they are successively treated in suitable coating equipment with aqueous solutions of crystallisable sugar such as sucrose or dextrose, which, depending on the stage of coating reached, may contain other functional ingredients, e.g. fillers, colors, etc.

In one presently preferred embodiment, the coating agent applied in a hard coating process is a sugarless coating agent, e.g. a polyol including as examples sorbitol, maltitol, mannitol, xylitol, erythritol, lactitol and isomalt or e.g. a mono- di-saccharide including as example trehalose. Or alternatively a sugar free soft coating prepared by alternately applying to the product surface a syrup of a polyol or a mono- di-saccharide, including as examples sorbitol, maltitol, mannitol, xylitol, erythritol, lactitol, isomalt or trehalose.

In further useful embodiments, a film coating is provided by film-forming agents such as a cellulose derivative, a modified starch, a dextrin, gelatine, zein, shellac, gum arabic, a vegetable gum, a synthetic polymer, etc. or a combination thereof.

In an embodiment of the invention, the outer coating comprises at least one additive component selected from the group comprising a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar and an acid.

A coated toffee gum product according to the invention may have any form, shape or dimension that permits the toffee gum to be coated using any conventional coating process. Accordingly, the product may be e.g. in a form selected from a pellet, a cushion-shaped pellet, a stick, a tablet, a chunk, a pastille, a pill, a ball and a sphere, and typically the weight of the uncoated product may be 0.5 to 10 grams.

The following non-limiting examples illustrate the manufacturing of a toffee gum confectionery product according to the invention. Resistance of the toffee gum substance towards fat and chocolate has been investigated.

### EXAMPLE 1

### Preparation of toffee gums

Toffee gum products were prepared having the toffee gum substance formulations of table 1.

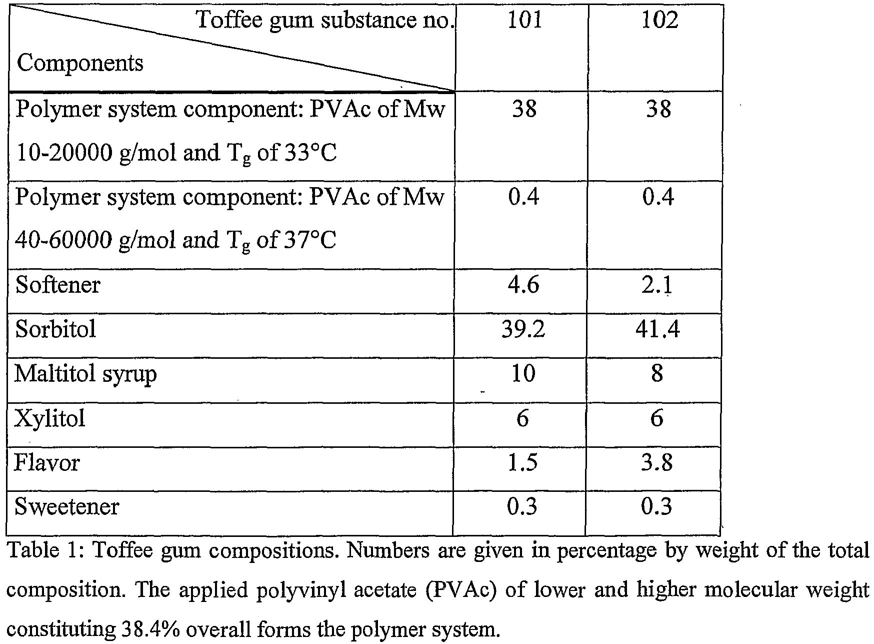

The preparation involved the following procedure: Polymer system and softeners were mixed in a mixer, such as e.g. a Z-blade mixing kettle. The mixing process was carried out at a temperature of about 40-80°C for a period of about 10-25 minutes, thereby preparing a homogeneous mixture of each polymer system. In case, one single polymer constitutes the polymer system, the mixing procedure serves yet to modify the polymer texture prior to the application in a toffee gum according to the invention. The mixing process, the heating, and a possible softening agent all contribute to a softening of the polymer system prior to mixing of the final toffee gum substance composition.

The mixed polymer system may be discharged from the mixer and allowed to cool to room temperature before transferring into a second mixer. Alternatively, the mixed polymer system may be transferred in its heated state into a second mixer, or the polymer system may be kept within the first mixer ready for addition of the further ingredients to prepare the toffee gum.

The polymer system and the remainder components are all mixed together in a mixer at a temperature of about 40-80°C. A mixing kettle, e.g. with horizontally placed Z-shaped arms may for example be used. Initially, the polymer system and about half of the sorbitol powder are mixed for about 3 minutes. At this time, the mixer should preferably be at the required temperature of 40-80°C. If necessary, the mixer may be preheated, e.g. for about 15 minutes. After mixing the polymer system and the first half portion of the sorbitol, the remaining half portion of sorbitol is added and mixed for 2 minutes, whereupon maltitol syrup is slowly added and mixing is continued for 5 minutes. Flavors are then added, and the composition is mixed for 3 minutes. Softener is slowly added and mixed for about 2 minutes. Then high-intensity sweeteners are added and mixed for 3 minutes. Xylitol is added and mixing continued for 3 minutes, whereupon the resulting toffee gum composition is discharged and e.g. transferred to a pan at a temperature of 40-60 °C. The product is then rolled and scored, punched or formed in another way into cores, sticks, balls, cubes, and any other desired shape, optionally followed by coating and polishing processes prior to packaging.

Evidently, within the scope of the invention, other processes and ingredients may be applied in the process of manufacturing the toffee gum.

### EXAMPLE 2

### Evaluation of resistance of toffee gum substance towards chocolate

Samples of 0.42 g toffee gum substance 101 of example 1 were each chewed with 3.32 g sugar-free chocolate for 4 minutes with a chewing rate of 1 chew/sec.

It was observed that that toffee gum substance was not dissolved, but maintained its toffee-like texture during chewing and thereby mixing with chocolate.

It was concluded that a ratio of 1 part of toffee gum substance to at least 8 parts of chocolate in the final toffee gum product is applicable without spoiling the toffee texture experienced by the consumer.

### EXAMPLE 3

### Evaluation of small chocolate-coated toffee gum pieces

Samples of 0.04g to 0.12g toffee gum substance 101 were coated by chocolate in an amount of about 2.3 times the weight of the toffee gum substance. The chocolate-coated toffee gums were chewed in portions of 3g, and it was observed that they were easily chewed to a coherent substance in the mouth. The toffee gum did not dissolve or loose its toffee texture.

It was concluded that the toffee gum of the invention may be prepared as small pieces, which are to be chewed together by the user, and furthermore the toffee gum pieces may comprise a considerable amount of chocolate without compromising the quality of the product or the desired toffee-like texture.

### EXAMPLE 4

### Evaluation of resistance of emulsifier-containing toffee gum substance towards chocolate

The evaluated toffee gum substance corresponded to formulation no. 102 of example 1 but comprised 5% by weight of emulsifier (mono- and di-glyceride), which substituted an equivalent percentage of sorbitol.

Samples of 1.5 g toffee gum substance were chewed with 3 g chocolate for 5 minutes, and it was observed that the toffee-texture was maintained throughout the chewing period. Thus, it was concluded that at least 5% by weight of emulsifier may be added to the toffee gum substance without compromising the desired texture, even when adding chocolate in an amount of 200% by weight of the toffee gum substance, corresponding to a content of chocolate in a final toffee gum product of about 66% by weight of the toffee gum product.

### EXAMPLE 5

### Evaluation of miscibility between toffee gum substance and cocoa butter and resistance of toffee gum substance towards fats

Toffee gum substance 101 according to example 1 was further mixed with cocoa butter during the mixing procedure. It was observed that cocoa butter was difficult to mix into the polymer system alone, but after addition of sorbitol the miscibility was improved. Altogether 120g cocoa butter was added to 1300g of toffee gum substance composition, and the texture of the obtained product was still maintained as a toffee-like texture according to the invention. The conclusion was that the toffee gum showed a quite large robustness towards the fat, i.e. cocoa butter, which was added.

### EXAMPLE 6

### Comparison of toffee gum and chewing gum by rheometric measurements

Toffee gum cores were prepared from toffee gum substance no. 101 of example 1 and compared to chewing gum samples with regard to rheological properties and thus the texture. Measurements were carried out by an AR 1000 rheometer from TA Instruments and at an oscillation torque of 10 µN·m and 37°C.

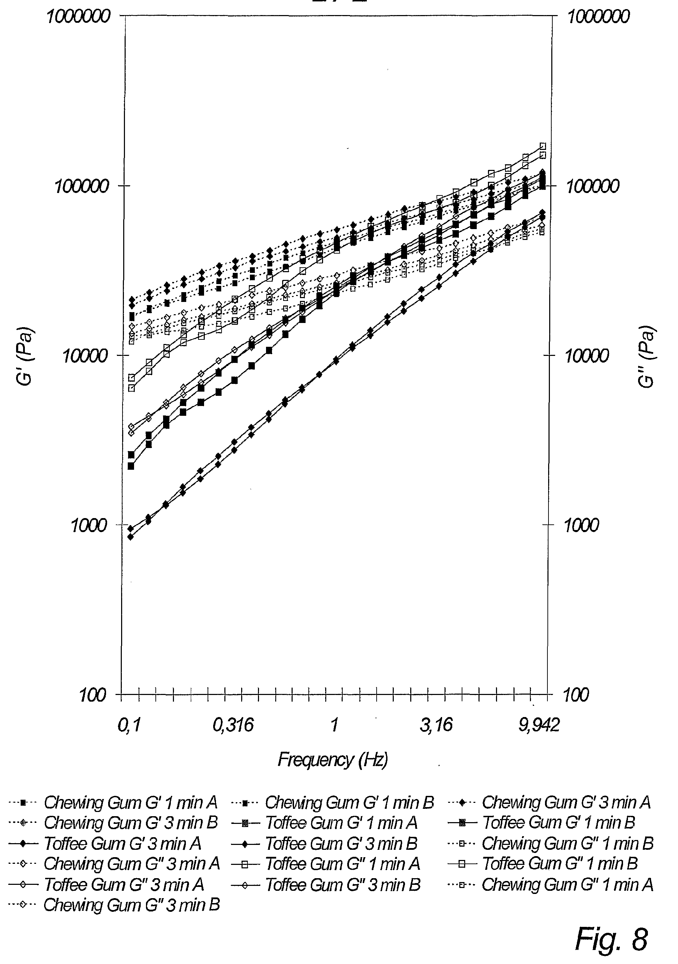

Results hereof are shown in fig. 8, which depicts rheometric measurements of storage modulus G' (Pa) and loss modulus G" (Pa) at increasing frequencies (Hz) for the tested samples of toffee gum and chewing gum. The tested samples included two samples of both toffee gum and chewing gum, which had been chewed for 1 minute, and likewise two samples of each product having been chewed for 3 minutes.

The conclusion when comparing toffee gum and chewing gum having been chewed for 1 minute are generally very similar to the conclusion of comparing samples having been chewed for 3 minutes.

A significant difference seen from the measurements is that for each sample of toffee gum, G" was larger than G', while the opposite relationship was found for chewing gum. This means that the toffee gum texture was dominated by G", i.e. the loss modulus, in contrast to chewing gum, which was dominated by G', i.e. the storage modulus. It is hereby evident that addition of elastomeric polymers, which increases the storage modulus, may destroy the desired toffee gum texture and cause a more chewing gum-like texture.

Looking at G' and G" independently, the following may be observed.

The slope of both G' and G" vs frequency is clearly more steep in the measurements of toffee gum as compared to chewing gum.

At all the measured frequencies from 0.1 up to about 5 Hz, G' was clearly lower for the toffee gum samples than for the chewing gum samples. Thus, the storage modulus G' was less pronounced for toffee gum compared to chewing gum. Moreover, it is seen that at frequencies from about 2 Hz and more, G" was higher for the toffee gum samples than for the chewing gum samples. Thus, at these frequencies, the loss modulus G" was more pronounced for toffee gum compared to chewing gum.

It is furthermore noted that G' of the toffee gum samples were lower for samples having been chewed for 3 minutes than for the samples having only been chewed for 1 minute. The same is seen for G".

In contrast, the opposite is noticed for the chewing gum samples, for which G' and G" were on a higher level for the samples chewed for 3 minutes compared to only 1 minute.

Summing up, it has been found that the desired textural properties of the toffee gum may be attained, when one or more of the following properties are obtained:
- the storage modulus G' is lower than the loss modulus G",
- the ratio of loss modulus G" to the storage modulus G' (i.e. G"/G) is in the range of 1 to 5,
- the storage modulus G' decreases during at least a part of a chewing process, and/or
- the loss modulus G" decreases during at least a part of a chewing process.

### EXAMPLE 7

Toffee gum cores were prepared from toffee gum substance no. 101 and coated by chocolate. The coated toffee gum cores were exposed to 70 RH at a temperature of 30°C and showed a remarkable stability against humidity. Increasing the thickness of the chocolate coating was found to increase the protective effect and thus provide increased stability when exposing the toffee gum to humidity in the surroundings.

## Claims

1. Toffee gum comprising a polymer system, and at least 1% by weight of chocolate,
said polymer system constituting at least 10% by weight of said toffee gum, at least 70% by weight of said polymer system comprising polyvinyl acetate,
said polyvinyl acetate having a glass transition temperature (T_{g}) in the range of 0 to 60 °C, and
said chocolate constituting at the most 90% by weight of said toffee gum.

2. Toffee gum according to claim 1, wherein said glass transition temperature (T_{g}) of
said polyvinyl acetate is in the range of 18 to 55 °C, preferably in the range of 25 to 40 °C.

3. Toffee gum according to any of the claims 1 or 2, wherein said toffee gum comprises at least one flavor and at least one sweetener.

4. Toffee gum according to any of the claims 1 - 3, wherein said polymer system constitutes at least 12% by weight of said toffee gum.

5. Toffee gum according to any of the claims 1 - 4, wherein said polymer system constitutes in the range of 15% to 95%, preferably 20% to 85%, more preferably 25% to 75%, and most preferably 30% to 65% by weight of said toffee gum.

6. Toffee gum according to any of the claims 1 - 5, wherein said polymer system comprises at least one substantially non-elastomeric polymer in an amount in the range of 70% to 100%, preferably 80% to 100% by weight of said polymer system.

7. Toffee gum according to any of the claims 1 - 6, wherein the polymer system is substantially formed by polyvinyl acetate(s) alone.

8. Toffee gum according to any of the claims 1 - 7, wherein said polyvinyl acetate has a molecular weight (Mw) in the range of 1000 to 250000 g/mol, preferably in the range of 2000 to 100000 g/mol, and most preferably in the range of 15000 to 60000 g/mol.

9. Toffee gum according to any of the claims 1 - 8, wherein the polymer system comprises at least one low-molecular weight polyvinyl acetate having a molecular weight (Mw) of 2000 to 40000 g/mol, and
at least one high-molecular weight PVAc having a molecular weight (Mw) of 40001 to 200000 g/mol.

10. Toffee gum according to any of the claims 1 - 9, wherein the polymer system comprises at least one low-molecular weight (Mw) polyvinyl acetate having a molecular weight of 9000 to 30000 g/mol, preferably 13000 to 21000 g/mol.

11. Toffee gum according to any of the claims 1 - 10, wherein the toffee gum comprises at least one low-molecular weight polyvinyl acetate having a molecular weight (Mw) of 2000 to 40000 g/mol in an amount of at least 70%, preferably at least 90% by weight of the polymer system.

12. Toffee gum according to any of the claims 1 - 11, wherein the toffee gum comprises at least one high-molecular weight polyvinyl acetate having a molecular weight (Mw) of 40000 to 100000 in an amount from 0.5 to 10 % by weight of the polymer system.

13. Toffee gum according to any of the claims 1 - 12, wherein at least 70%, preferably at least 90%, and most preferably 100% by weight of said polymer system is polyvinyl acetate having a glass transition temperature (T_{g}) in the range of 0 to 60 °C, preferably 10 to 50 °C, and most preferably 20 to 40°C.

14. Toffee gum according to any of the claims 1 - 13, wherein at the most 30%, preferably at the most 10% by weight of said polymer system is polyvinyl acetate having a molecular weight (Mw) in the range of 40001 - 200000 g/mol.

15. Toffee gum according to any of the claims 1 - 14, wherein said polymer system comprises less than 4% by weight of polymers having a molecular weight (Mw) from 50000 to 99999 g/mol.

16. Toffee gum according to any of the claims 1 - 15, wherein said polymer system comprises less than 2% by weight of polymers having a molecular weight (Mw) from 100000 to 199999 g/mol.

17. Toffee gum according to any of the claims 1 - 16, wherein said polymer system comprises less than 1% by weight of polymers having a molecular weight (Mw) from 200000 to 399999 g/mol.

18. Toffee gum according to any of the claims 1 - 17, wherein said polymer system comprises less than 0.5% by weight of polymers having a molecular weight (Mw) from 399000 to 800000 g/mol.

19. Toffee gum according to any of the claims 1 - 18, wherein said polymer system is substantially free of elastomers.

20. Toffee gum according to any of the claims 1 - 19, wherein said toffee gum is **characterised by** having an storage modulus G' in the range of 100 Pa to 150000 Pa as measured at a frequency within the range of 0.1 to 10 Hz.

21. Toffee gum according to any of the claims 1 - 20, wherein said toffee gum is **characterised by** having a loss modulus G" in the range of 1000 Pa to 200000 Pa as measured at a frequency within the range of 0.1 to 10 Hz.

22. Toffee gum according to any of the claims 1 - 21, wherein said toffee gum is **characterised by** having a ratio of said loss modulus G" to said storage modulus G' in the range of 1 to 5.

23. Toffee gum according to any of the claims 1 - 22, wherein said storage modulus G' is lower than said loss modulus G".

24. Toffee gum according to any of the claims 1 - 23, wherein said storage modulus G' is lower than said loss modulus G" as measured at a frequency of 1 Hz.

25. Toffee gum according to any of the claims I - 24, wherein said loss modulus G" and said storage modulus G' are both lower than 10000 Pa as measured at a frequency of 0.1 Hz.

26. Toffee gum according to any of the claims 1 - 25, wherein said loss modulus G" is higher than 10000 Pa as measured at a frequency of 1 Hz.

27. Toffee gum according to any of the claims 1 - 26, wherein said storage modulus G' is lower than 40000 Pa, preferably lower than 30000 Pa as measured at a frequency of 1 Hz.

28. Toffee gum according to any of the claims 1 - 27, wherein said storage modulus G' decreases during at least a part of a chewing process.

29. Toffee gum according to any of the claims 1 - 28, wherein said storage modulus G' decreases in the chewing process during the first 1 to 3 minutes.

30. Toffee gum according to any of the claims 1 - 29, wherein said loss modulus G" decreases during at least a part of the chewing process.

31. Toffee gum according to any of the claims 1 - 30, wherein said loss modulus G" decreases in the chewing process during the first 1 to 3 minutes.

32. Toffee gum according to any of the claims 1 - 31, wherein said chewing process involves a chewing frequency at 1 Hz.

33. Toffee gum according to any of the claims 1 - 32, wherein said storage modulus G' and said loss modulus G" are measured at an oscillation torque of 8 to 12 µN·m.

34. Toffee gum according to any of the claims 1 - 33, wherein said toffee gum comprises a toffee gum substance being formed from a mixture of said polymer system with at least one sweetener and at least one flavor.

35. Toffee gum according to any of the claims 1 - 34, wherein said toffee gum comprises toffee or caramel in an amount in the range of 1% to 85% by weight of said toffee gum.

36. Toffee gum according to any of the claims 1 - 35, wherein at least a part of said chocolate is forming or comprised in at least one chocolate module of said toffee gum.

37. Toffee gum according to any of the claims 1 - 36, wherein said at least one chocolate module comprises further ingredients.

38. Toffee gum according to any of the claims 1 - 37, wherein at least one of said at least one chocolate module is forming an outer coating of said toffee gum.

39. Toffee gum according to any of the claims 1 - 38, wherein said toffee gum substance is forming a core and at least one of said at least one chocolate module is situated in said core.

40. Toffee gum according to any of the claims 1 - 39, wherein said core comprises several of said at least one chocolate module.

41. Toffee gum according to any of the claims 1 - 40, wherein said chocolate is applied in an amount in the range of 5% to 80%, preferably 10% to 60% by weight of said toffee gum.

42. Toffee gum according to any of the claims 1 - 41, wherein said chocolate contains sugar.

43. Toffee gum according to any of the claims 1 - 42, wherein said chocolate is substantially sugar-free.

44. Toffee gum according to any of the claims 1 - 43, wherein said chocolate comprises cocoa butter in an amount of 15% to 90% by weight of said chocolate.

45. Toffee gum according to any of the claims 1 - 44, wherein said chocolate is selected from the group consisting of dark chocolate, milk, and white chocolate, or any combination thereof.

46. Toffee gum according to any of the claims 1 - 45, wherein said chocolate is selected from the group consisting of unsweetened chocolate, bittersweet chocolate, semisweet chocolate, and sweet chocolate, or any combination thereof.

47. Toffee gum according to any of the claims 1 - 46, wherein said chocolate is chocolate couverture.

48. Toffee gum according to any of the claims 1 - 47, wherein said chocolate is a compound chocolate.

49. Toffee gum according to any of the claims 1 - 48, wherein at least a part of said cocoa butter in said chocolate is replaced by an alternative vegetable fat or oil.

50. Toffee gum according to any of the claims 1 - 49, wherein said chocolate is substantially free of fat.

51. Toffee gum according to any of the claims 1 - 50, wherein said chocolate is selected from the group consisting of liquid chocolate, solid chocolate, and powdered chocolate, or any combination thereof.

52. Toffee gum according to any of the claims 1 - 51, wherein said chocolate module forming an outer coating has been applied by a coating method selected from the group consisting of spray coating, pan coating, curtain coating, or any combination thereof.

53. Toffee gum according to any of the claims 1 - 52, wherein said chocolate is substituted by a chocolate substitute.

54. Toffee gum according to any of the claims 1 - 53, wherein said toffee gum comprises a precoat below said outer coating.

55. Toffee gum according to any of the claims 1 - 54, wherein said outer coating is supplied with a polishing.

56. Toffee gum according to any of the claims 1 - 55, wherein said toffee gum is combined with further ingredients selected from the group consisting of coconut, rice, maize, nougat, marzipan, macaroon, almonds, fruits, berries, caramel, liquor, nuts, hazelnuts, peanuts, walnuts, liqourice, wine gum, or any combination thereof.

57. Toffee gum according to any of the claims 1 - 56, wherein said toffee gum comprises at least one softener in an amount in the range of 0.5% to 25%, preferably in the range of 1% to 18%, and most preferably in the range or 2% to 12% by weight of said toffee gum.

58. Toffee gum according to any of the claims 1 - 57, wherein said at least one softener is selected from the group consisting of triacetin, lecithin, acetylated glycerides, mono- and di-glycerides, or any combination thereof.

59. Toffee gum according to any of the claims 1 - 58, wherein said toffee gum comprises at least one syrup of a polyol in an amount of 1% to 25%, preferably 5% to 20% by weight of said toffee gum.

60. Toffee gum according to any of the claims 1 - 59, wherein said polymer system comprises plasticizer(s).

61. Toffee gum according to any of the claims 1 - 60, wherein the toffee gum comprises less than 6%, preferably less than 4% by weight of filler, preferably less than 1% by weight.

62. Toffee gum according to any of the claims 1 - 61, wherein the toffee gum is substantially free of filler.

63. Toffee gum according to any of the claims 1 - 62, wherein said toffee gum comprises at said least one flavor in an amount of 0.001% to 30% by weight, and said at least one sweetener in an amount of 5% to 80% by weight.

64. Toffee gum according to any of the claims 1 - 63, wherein said flavor is a caramel flavor comprising an amount in the range of 1% to 30% by weight of said toffee gum substance.

65. Toffee gum according to any of the claims 1 - 64, wherein said at least one flavor is substantially oil-based and/or substantially hydrophilic.

66. Toffee gum according to any of the claims 1 - 65, wherein said at least one sweetener comprises sugar.

67. Toffee gum according to any of the claims 1 - 66, wherein said sugar is selected from the group consisting of mono-, di-, or polysaccharides and any combination thereof.

68. Toffee gum according to any of the claims 1 - 67, wherein said sugar is selected from the group consisting of sucrose, dextrose, maltose, dextrins, trehalose, tagatose, dried invert sugar, fructose, levulose, galactose, corn syrup solids, polydextroseII, polydextrose ultra, incline, or any combination thereof.

69. Toffee gum according to any of the claims 1 - 68, wherein said at least one sweetener is an artificial sweetener.

70. Toffee gum according to any of the claims 1 - 69, wherein said artificial sweetener is selected from the group consisting of sorbitol, mannitol, maltitol, xylitol, erythritol, lactitol, isomalt, derivatives of isomalt, or any combination thereof.

71. Toffee gum according to any of the claims 1 - 70, wherein said artificial sweetener is a high-intensity artificial sweetener selected from the group consisting of aspartame, salts of acesulfame, alitame, neotame, twinsweet, saccharin and its salts, cyclamic acid and its salts, glycyrrhizin, dihydrochalcones, thaumatin, monellin, stevioside, sucralose, or any combination thereof.

72. Toffee gum according to any of the claims 1 - 71, wherein said toffee gum further comprises active ingredients.

73. Toffee gum according to any of the claims 1 - 72, wherein said toffee gum forms a tablet comprising a chewable polymer system.

74. Toffee gum according to any of the claims 1 - 73, wherein said toffee gum is formed in shapes such as cores, ellipsoid, balls, cylinders, squares, rectangular, hexagonal, strips, paraboloid, donut formed, ring formed, teddy bear formed and/or multi-modular.

75. Toffee gum according to any of the claims 1 - 74, wherein said toffee gum weighs from ¼ gram to 10 grams.

76. Toffee gum according to any of the claims 1 - 75, wherein said toffee gum weighs from ½ gram to 5 grams.

77. Toffee gum according to any of the claims 1 - 76, wherein said toffee gum is provided with an outer coating.

78. Toffee gum according to any of the claims 1 - 77, wherein said coating is selected from the group consisting of hard coating, soft coating and edible film-coating.

79. Toffee gum according to any of the claims 1 - 78, wherein said toffee gum is center-filled.

80. Toffee gum according to any of the claims 1 - 79, wherein said toffee gum is compressed.

81. Method of manufacturing a toffee gum according to any of the claims 1 - 80, whereby the product is manufactured by a batch process or an extruder process.

82. Method of manufacturing a toffee gum according to claim 81 with a coating of chocolate, said method comprising the steps of applying chocolate in liquid form to the surface of a toffee gum core and subjecting the chocolate coated toffee gum to a temperature, which causes the liquid chocolate to harden.

## Patentansprüche

1. Toffeegummi, umfassend ein Polymersystem und mindestens 1 Gew.-% Schokolade, wobei das Polymersystem mindestens 10 Gew.-% des Toffeegummis ausmacht, wobei mindestens 70 Gew.-% des Polymersystems Polyvinylacetat umfassen, wobei das Polyvinylacetat eine Glasübergangstemperatur (T_{g}) im Bereich von 0 bis 60°C aufweist, und die Schokolade höchstens 90 Gew.-% des Toffeegummis ausmacht.

2. Toffeegummi nach Anspruch 1, wobei die Glasübergangstemperatur (Tg) des Polyvinylacetats im Bereich von 18 bis 55°C, vorzugsweise im Bereich von 25 bis 40°C liegt.

3. Toffeegummi nach einem der Ansprüche 1 oder 2, wobei der Toffeegummi mindestens einen Geschmack- oder Aromastoff und mindestens ein Süßungsmittel umfasst.

4. Toffeegummi nach einem der Ansprüche 1-3, wobei das Polymersystem mindestens 12 Gew.-% des Toffeegummis ausmacht.

5. Toffeegummi nach einem der Ansprüche 1-4, wobei das Polymersystem zwischen 15 Gew.-% und 95 Gew.-%, vorzugsweise 20 Gew.-% bis 85 Gew.-%, mehr bevorzugt 25 Gew.-% bis 75 Gew.-% und am meisten bevorzugt 30 Gew.-% bis 65 Gew.-% des Toffeegummis ausmacht.

6. Toffeegummi nach einem der Ansprüche 1-5, wobei das Polymersystem mindestens ein im Wesentlichen nicht-elastomeres Polymer in einer Menge im Bereich von 70 Gew.-% bis 100 Gew.-%, vorzugsweise 80 Gew.-% bis 100 Gew.-% des Polymersystems umfasst.

7. Toffeegummi nach einem der Ansprüche 1-6, wobei das Polymersystem im Wesentlichen aus Polyvinylacetat(en) allein gebildet wird.

8. Toffeegummi nach einem der Ansprüche 1-7, wobei das Polyvinylacetat ein Molekulargewicht (Mw) im Bereich von 1000 bis 250000 g/mol, vorzugsweise im Bereich von 2000 bis 100000 g/mol und am meisten bevorzugt im Bereich von 15000 bis 60000 g/mol aufweist.

9. Toffeegummi nach einem der Ansprüche 1-8, wobei das Polymersystem mindestens ein ein geringes Molekulargewicht aufweisendes Polyvinylacetat mit einem Molekulargewicht (Mw) von 2000 bis 40000 g/mol und
mindestens ein ein hohes Molekulargewicht aufweisendes PVAc mit einem Molekulargewicht (Mw) von 40001 bis 200000 g/mol umfasst.

10. Toffeegummi nach einem der Ansprüche 1-9, wobei das Polymersystem mindestens ein ein geringes Molekulargewicht (Mw) aufweisendes Polyvinylacetat mit einem Molekulargewicht von 9000 bis 30000 g/mol, vorzugsweise 13000 bis 21000 g/mol umfasst.

11. Toffeegummi nach einem der Ansprüche 1-10, wobei der Toffeegummi mindestens ein ein geringes Molekulargewicht aufweisendes Polyvinylacetat mit einem Molekulargewicht (Mw) von 2000 bis 40000 g/mol in einer Menge von mindestens 70 Gew.-%, vorzugsweise mindestens 90 Gew.-% des Polymersystems umfasst.

12. Toffeegummi nach einem der Ansprüche 1-11, wobei der Toffeegummi mindestens ein ein hohes Molekulargewicht aufweisendes Polyvinylacetat mit einem Molekulargewicht (Mw) von 40000 bis 100000 g/mol in einer Menge von 0,5 bis 10 Gew.-% des Polymersystems umfasst.

13. Toffeegummi nach einem der Ansprüche 1-12, wobei mindestens 70 Gew.-%, vorzugsweise mindestens 90 Gew.-% und am meisten bevorzugt 100 Gew.-% des Polymersystems Polyvinylacetat mit einer Glasübergangstemperatur (T_{g}) im Bereich von 0 bis 60°C, vorzugsweise 10 bis 50°C und am meisten bevorzugt 20 bis 40°C sind.

14. Toffeegummi nach einem der Ansprüche 1-13, wobei höchstens 30 Gew.-%, vorzugsweise höchstens 10 Gew.-% des Polymersystems Polyvinylacetat mit einem Molekulargewicht (Mw) im Bereich von 40001 - 200000 g/mol sind.

15. Toffeegummi nach einem der Ansprüche 1-14, wobei das Polymersystem weniger als 4 Gew.-% Polymere mit einem Molekulargewicht (Mw) von 50000 bis 99999 g/mol umfasst.

16. Toffeegummi nach einem der Ansprüche 1-15, wobei das Polymersystem weniger als 2 Gew.-% Polymere mit einem Molekulargewicht (Mw) von 100000 bis 199999 g/mol umfasst.

17. Toffeegummi nach einem der Ansprüche 1-16, wobei das Polymersystem weniger als 1 Gew.-% Polymere mit einem Molekulargewicht (Mw) von 200000 bis 399999 g/mol umfasst.

18. Toffeegummi nach einem der Ansprüche 1-17, wobei das Polymersystem weniger als 0,5 Gew.-% Polymere mit einem Molekulargewicht (Mw) von 399000 bis 800000 g/mol umfasst.

19. Toffeegummi nach einem der Ansprüche 1-18, wobei das Polymersystem im Wesentlichen frei von Elastomeren ist.

20. Toffeegummi nach einem der Ansprüche 1-19, wobei der Toffeegummi **dadurch gekennzeichnet ist, dass** er einen Speichermodul G' im Bereich von 100 Pa bis 150000 Pa aufweist, wenn bei einer Frequenz innerhalb des Bereichs von 0,1 bis 10 Hz gemessen wird.

21. Toffeegummi nach einem der Ansprüche 1-20, wobei der Toffeegummi **dadurch gekennzeichnet ist, dass** er einen Verlustmodul G" im Bereich von 1000 Pa bis 200000 Pa aufweist, wenn bei einer Frequenz innerhalb des Bereichs von 0,1 bis 10 Hz gemessen wird.

22. Toffeegummi nach einem der Ansprüche 1-21, wobei der Toffeegummi **dadurch gekennzeichnet ist, dass** er ein Verhältnis von dem Verlustmodul G" zu dem Speichermodul G' im Bereich von 1 bis 5 aufweist.

23. Toffeegummi nach einem der Ansprüche 1-22, wobei der Speichermodul G' niedriger als der Verlustmodul G" ist.

24. Toffeegummi nach einem der Ansprüche 1-23, wobei der Speichermodul G' niedriger als der Verlustmodul G" ist, wenn bei einer Frequenz von 1 Hz gemessen wird.

25. Toffeegummi nach einem der Ansprüche 1-24, wobei der Verlustmodul G" und der Speichermodul G' beide niedriger als 10000 Pa sind, wenn bei einer Frequenz von 0,1 Hz gemessen wird.

26. Toffeegummi nach einem der Ansprüche 1-25, wobei der Verlustmodul G" höher als 10000 Pa ist, wenn bei einer Frequenz von 1 Hz gemessen wird.

27. Toffeegummi nach einem der Ansprüche 1-26, wobei der Speichermodul G' niedriger als 40000 Pa, vorzugsweise niedriger als 30000 Pa ist, wenn bei einer Frequenz von 1 Hz gemessen wird.

28. Toffeegummi nach einem der Ansprüche 1-27, wobei der Speichermodul G' während mindestens eines Teils eines Kauprozesses abnimmt.

29. Toffeegummi nach einem der Ansprüche 1-28, wobei der Speichermodul G' in dem Kauprozess während der ersten 1 bis 3 Minuten abnimmt.

30. Toffeegummi nach einem der Ansprüche 1-29, wobei der Verlustmodul G" während mindestens eines Teils des Kauprozesses abnimmt.

31. Toffeegummi nach einem der Ansprüche 1-30, wobei der Verlustmodul G" in dem Kauprozess während der ersten 1 bis 3 Minuten abnimmt.

32. Toffeegummi nach einem der Ansprüche 1-31, wobei der Kauprozess eine Kaufrequenz bei 1 Hz umfasst.

33. Toffeegummi nach einem der Ansprüche 1-32, wobei der Speichermodul G' und der Verlustmodul G" bei einem Oszillationsdrehmoment von 8 bis 12 µN · m gemessen werden.

34. Toffeegummi nach einem der Ansprüche 1-33, wobei der Toffeegummi eine Toffeegummisubstanz, die aus einer Mischung von dem Polymersystem mit mindestens einem Süßungsmittel und mindestens einem Geschmack- oder Aromastoff gebildet wird, umfasst.

35. Toffeegummi nach einem der Ansprüche 1-34, wobei der Toffeegummi Toffee oder Karamell in einer Menge im Bereich von 1 Gew.-% bis 85 Gew.-% des Toffeegummis umfasst.

36. Toffeegummi nach einem der Ansprüche 1-35, wobei mindestens ein Teil der Schokolade mindestens ein Schokolademodul des Toffeegummis bildet oder darin enthalten ist.

37. Toffeegummi nach einem der Ansprüche 1-36, wobei das mindestens eine Schokolademodul weitere Bestandteile umfasst.

38. Toffeegummi nach einem der Ansprüche 1-37, wobei mindestens eines von dem mindestens einen Schokolademodul einen äußeren Überzug des Toffeegummis bildet.

39. Toffeegummi nach einem der Ansprüche 1-38, wobei die Toffeegummisubstanz einen Kern bildet und mindestens eines von dem mindestens einen Schokolademodul sich in dem Kern befindet.

40. Toffeegummi nach einem der Ansprüche 1-39, wobei der Kern mehrere von dem mindestens einen Schokolademodul umfasst.

41. Toffeegummi nach einem der Ansprüche 1-40, wobei die Schokolade in einer Menge im Bereich von 5 Gew.-% bis 80 Gew.-%, vorzugsweise 10 Gew.-% bis 60 Gew.-% des Toffeegummis angewendet wird.

42. Toffeegummi nach einem der Ansprüche 1-41, wobei die Schokolade Zucker enthält.

43. Toffeegummi nach einem der Ansprüche 1-42, wobei die Schokolade im Wesentlichen zuckerfrei ist.

44. Toffeegummi nach einem der Ansprüche 1-43, wobei die Schokolade Kakaobutter in einer Menge von 15 Gew.-% bis 90 Gew.-% der Schokolade umfasst.

45. Toffeegummi nach einem der Ansprüche 1-44, wobei die Schokolade aus der Gruppe bestehend aus dunkler Schokolade, Milchschokolade und weißer Schokolade oder einer jeglichen Kombination davon ausgewählt ist.

46. Toffeegummi nach einem der Ansprüche 1-45, wobei die Schokolade aus der Gruppe bestehend aus ungesüßter Schokolade, bittersüßer Schokolade, halbsüßer Schokolade und süßer Schokolade oder einer jeglichen Kombination davon ausgewählt ist.

47. Toffeegummi nach einem der Ansprüche 1-46, wobei die Schokolade Schokoladenkuvertüre ist.

48. Toffeegummi nach einem der Ansprüche 1-47, wobei die Schokolade ein 'compound chocolate'-Schokoladenerzeugnis ist.

49. Toffeegummi nach einem der Ansprüche 1-48, wobei mindestens ein Teil der Kakaobutter in der Schokolade durch ein alternatives pflanzliches Fett oder Öl ersetzt ist.

50. Toffeegummi nach einem der Ansprüche 1-49, wobei die Schokolade im Wesentlichen frei von Fett ist.

51. Toffeegummi nach einem der Ansprüche 1-50, wobei die Schokolade aus der Gruppe bestehend aus flüssiger Schokolade, fester Schokolade und pulverförmiger Schokolade oder einer jeglichen Kombination davon ausgewählt ist.

52. Toffeegummi nach einem der Ansprüche 1-51, wobei das Schokolademodul, welches einen äußeren Überzug bildet, durch ein Beschichtungsverfahren, ausgewählt aus der Gruppe bestehend aus Sprühbeschichten, Trommelbeschichten, Vorhangbeschichten oder einer jeglichen Kombination davon, aufgebracht worden ist.

53. Toffeegummi nach einem der Ansprüche 1-52, in welchem die Schokolade durch einen Schokoladenersatz ersetzt ist.

54. Toffeegummi nach einem der Ansprüche 1-53, wobei der Toffeegummi eine Precoat-Schicht unter dem äußeren Überzug umfasst.

55. Toffeegummi nach einem der Ansprüche 1-54, wobei der äußere Überzug mit einer Politur versehen ist.

56. Toffeegummi nach einem der Ansprüche 1-55, wobei der Toffeegummi mit weiteren Bestandteilen, ausgewählt aus der Gruppe bestehend aus Kokosnuss, Reis, Mais, Nougat, Marzipan, Makrone, Mandeln, Früchten, Beeren, Karamell, Spirituosen, Nüssen, Haselnüssen, Erdnüssen, Walnüssen, Süßholz, Weingummi oder einer jeglichen Kombination davon, kombiniert ist.

57. Toffeegummi nach einem der Ansprüche 1-56, wobei der Toffeegummi mindestens ein Erweichungsmittel in einer Menge im Bereich von 0,5 Gew.-% bis 25 Gew.-%, vorzugsweise im Bereich von 1 Gew.-% bis 18 Gew.-% und am meisten bevorzugt im Bereich von 2 Gew.-% bis 12 Gew.-% des Toffeegummis umfasst.

58. Toffeegummi nach einem der Ansprüche 1-57, wobei das mindestens eine Erweichungsmittel aus der Gruppe bestehend aus Triacetin, Lecithin, acetylierten Glyceriden, Mono- und Diglyceriden oder einer jeglichen Kombination davon ausgewählt ist.

59. Toffeegummi nach einem der Ansprüche 1-58, wobei der Toffeegummi mindestens einen Sirup eines Polyols in einer Menge von 1 Gew.-% bis 25 Gew.-%, vorzugsweise 5 Gew.-% bis 20 Gew.-% des Toffeegummis umfasst.

60. Toffeegummi nach einem der Ansprüche 1-59, wobei das Polymersystem einen oder mehrere Weichmacher umfasst.

61. Toffeegummi nach einem der Ansprüche 1-60, wobei der Toffeegummi weniger als 6 Gew.-%, vorzugsweise weniger als 4 Gew.-% Füllstoff, vorzugsweise weniger als 1 Gew.-% umfasst.

62. Toffeegummi nach einem der Ansprüche 1-61, wobei der Toffeegummi im Wesentlichen frei von Füllstoff ist.

63. Toffeegummi nach einem der Ansprüche 1-62, wobei der Toffeegummi den mindestens einen Geschmack- oder Aromastoff in einer Menge von 0,001 Gew.-% bis 30 Gew.-% und das mindestens eine Süßungsmittel in einer Menge von 5 Gew.-% bis 80 Gew.-% umfasst.

64. Toffeegummi nach einem der Ansprüche 1-63, wobei der Geschmack- oder Aromastoff ein Karamellaroma, welches eine Menge im Bereich von 1 Gew.-% bis 30 Gew.-% der Toffeegummisubstanz umfasst, ist.

65. Toffeegummi nach einem der Ansprüche 1-64, wobei der mindestens eine Geschmack- oder Aromastoff im Wesentlichen Öl-basiert und/oder im Wesentlichen hydrophil ist.

66. Toffeegummi nach einem der Ansprüche 1-65, wobei das mindestens eine Süßungsmittel Zucker umfasst.

67. Toffeegummi nach einem der Ansprüche 1-66, wobei der Zucker aus der Gruppe bestehend aus Mono-, Di- oder Polysacchariden und einer jeglichen Kombination davon ausgewählt ist.

68. Toffeegummi nach einem der Ansprüche 1-67, wobei der Zucker aus der Gruppe bestehend aus Sucrose, Dextrose, Maltose, Dextrinen, Trehalose, Tagatose, getrocknetem Invertzucker, Fructose, Levulose, Galactose, Mais-Stärkezuckersirup-Feststoffen, Polydextrose II, Polydextrose ultra, Inulin oder einer jeglichen Kombination davon ausgewählt ist.

69. Toffeegummi nach einem der Ansprüche 1-68, wobei das mindestens eine Süßungsmittel ein künstlicher Süßstoff ist.

70. Toffeegummi nach einem der Ansprüche 1-69, wobei der künstliche Süßstoff aus der Gruppe bestehend aus Sorbitol, Mannitol, Maltitol, Xylitol, Erythritol, Lactitol, Isomalt, Derivaten von Isomalt oder einer jeglichen Kombination davon ausgewählt ist.

71. Toffeegummi nach einem der Ansprüche 1-70, wobei der künstliche Süßstoff ein hochintensiver künstlicher Süßstoff, ausgewählt aus der Gruppe bestehend aus Aspartam, Salzen von Acesulfam, Alitam, Neotam, Twinsweet, Saccharin und dessen Salzen, Cyclaminsäure und deren Salzen, Glycyrrhizin, Dihydrochalkonen, Thaumatin, Monellin, Steviosid, Sucralose oder einer jeglichen Kombination, ist.

72. Toffeegummi nach einem der Ansprüche 1-71, wobei der Toffeegummi des Weiteren Wirkstoffe umfasst.

73. Toffeegummi nach einem der Ansprüche 1-72, wobei der Toffeegummi eine Tablette, welche ein kaubares Polymersystem umfasst, bildet.

74. Toffeegummi nach einem der Ansprüche 1-73, wobei der Toffeegummi zu Gestalten wie Kernen, Ellipsoid, Bällen, Zylindern, Quadraten, Rechtecken, hexagonal, Streifen, Paraboloid, Donut-förmig, ringförmig, Teddybär-förmig und/oder multimodular geformt ist.

75. Toffeegummi nach einem der Ansprüche 1-74, wobei der Toffeegummi von ¼ Gramm bis 10 Gramm wiegt.

76. Toffeegummi nach einem der Ansprüche 1-75, wobei der Toffeegummi von ½ Gramm bis 5 Gramm wiegt.

77. Toffeegummi nach einem der Ansprüche 1-76, wobei der Toffeegummi mit einem äußeren Überzug versehen ist.

78. Toffeegummi nach einem der Ansprüche 1-77, wobei der Überzug aus der Gruppe bestehend aus harten Überzügen, weichen Überzügen und essbaren Filmüberzügen ausgewählt ist.

79. Toffeegummi nach einem der Ansprüche 1-78, wobei der Toffeegummi im Zentrum gefüllt ist.

80. Toffeegummi nach einem der Ansprüche 1-79, wobei der Toffeegummi verdichtet ist.

81. Verfahren zum Herstellen eines Toffeegummis nach einem der Ansprüche 1-80, durch welches das Produkt durch ein chargenweise ausgeführtes Verfahren oder ein Extruderverfahren hergestellt wird.

82. Verfahren zum Herstellen eines Toffeegummis nach Anspruch 81 mit einem Überzug aus Schokolade, wobei das Verfahren die Schritte umfasst, Schokolade in flüssiger Form auf die Oberfläche eines Toffeegummikerns aufzubringen und den mit Schokolade überzogenen Toffeegummi einer Temperatur auszusetzen, welche bewirkt, dass die flüssige Schokolade erhärtet.

## Revendications

1. Gomme de toffee comprenant un système polymère, et au moins 1 % en poids de chocolat, ledit système polymère constituant au moins 10 % en poids dudit gomme de toffee, au moins 70 % en poids dudit système polymère comprenant de l'acétate de polyvinyle, ledit acétate de polyvinyle ayant une température de transition vitreuse (T_{g}) comprise dans la plage allant de 0 à 60 °C, et ledit chocolat constituant au maximum 90 % en poids dudit gomme de toffee.

2. Gomme de toffee selon la revendication 1, dans lequel ladite température de transition vitreuse (T_{g}) dudit acétate de polyvinyle est comprise dans la plage allant de 18 à 55 °C, de préférence dans la plage allant de 25 à 40 °C.

3. Gomme de toffee selon l'une quelconque des revendications 1 ou 2, dans lequel ledit gomme de toffee comprend au moins un arôme et au moins un édulcorant.

4. Gomme de toffee selon l'une quelconque des revendications 1 à 3, dans lequel ledit système polymère constitue au moins 12 % en poids dudit gomme de toffee.

5. Gomme de toffee selon l'une quelconque des revendications 1 à 4, dans lequel ledit système polymère constitue de 15 % à 95 %, de préférence de 20 % à 85 %, plus préférablement de 25 % à 75 %, et de manière préférée entre toutes de 30 % à 65 % en poids dudit gomme de toffee.

6. Gomme de toffee selon l'une quelconque des revendications 1 à 5, dans lequel ledit système polymère comprend au moins un polymère sensiblement non-élastomère en quantité comprise dans la plage allant de 70 % à 100 %, de préférence de 80 % à 100 % en poids dudit système polymère.

7. Gomme de toffee selon l'une quelconque des revendications 1 à 6, dans lequel le système polymère est sensiblement formé par un/des acétate(s) de polyvinyle seul(s).

8. Gomme de toffee selon l'une quelconque des revendications 1 à 7, dans lequel ledit acétate de polyvinyle a un poids moléculaire (Mw) compris dans la plage allant de 1000 à 250 000 g/mol, de préférence dans la plage allant de 2000 à 100 000 g/mol, et de manière préférée entre toutes dans la plage allant de 15 000 à 60 000 g/mol.

9. Gomme de toffee selon l'une quelconque des revendications 1 à 8, dans lequel le système polymère comprend au moins un acétate de polyvinyle à faible poids moléculaire ayant un poids moléculaire (Mw) allant de 2000 à 40 000 g/mol, et au moins un PVAc à poids moléculaire élevé ayant un poids moléculaire (Mw) allant de 40 001 à 200 000 g/mol.

10. Gomme de toffee selon l'une quelconque des revendications 1 à 9, dans lequel le système polymère comprend au moins un acétate de polyvinyle à faible poids moléculaire (Mw) ayant un poids moléculaire allant de 9000 à 30 000 g/mol, de préférence de 13 000 à 21 000 g/mol.

11. Gomme de toffee selon l'une quelconque des revendications 1 à 10, dans lequel le gomme de toffee comprend au moins un acétate de polyvinyle à faible poids moléculaire ayant un poids moléculaire (Mw) allant de 2000 à 40 000 g/mol en quantité d'au moins 70 %, de préférence d'au moins 90 % en poids du système polymère.

12. Gomme de toffee selon l'une quelconque des revendications 1 à 11, dans lequel le gomme de toffee comprend au moins un acétate de polyvinyle à poids moléculaire élevé ayant un poids moléculaire (Mw) allant de 40 000 à 100 000 en quantité allant de 0,5 à 10 % en poids du système polymère.

13. Gomme de toffee selon l'une quelconque des revendications 1 à 12, dans lequel au moins 70 %, de préférence au moins 90 %, et de manière préférée entre toutes 100 % en poids dudit système polymère est de l'acétate de polyvinyle ayant une température de transition vitreuse (T_{g}) comprise dans la plage allant de 0 à 60°C, de préférence de 10 à 50 °C, et de manière préférée entre toutes de 20 à 40°C.

14. Gomme de toffee selon l'une quelconque des revendications 1 à 13, dans lequel au maximum 30 %, de préférence au maximum 10 % en poids dudit système polymère est de l'acétate de polyvinyle ayant un poids moléculaire (Mw) compris dans la plage allant de 40 001 à 200 000 g/mol.

15. Gomme de toffee selon l'une quelconque des revendications 1 à 14, dans lequel ledit système polymère comprend moins de 4 % en poids de polymères ayant un poids moléculaire (Mw) allant de 50 000 à 99 999 g/mol.

16. Gomme de toffee selon l'une quelconque des revendications 1 à 15, dans lequel ledit système polymère comprend moins de 2 % en poids de polymères ayant un poids moléculaire (Mw) allant de 100 000 à 199 999 g/mol.

17. Gomme de toffee selon l'une quelconque des revendications 1 à 16, dans lequel ledit système polymère comprend moins de 1 % en poids de polymères ayant un poids moléculaire (Mw) allant de 200 000 à 399 999 g/mol.

18. Gomme de toffee selon l'une quelconque des revendications 1 à 17, dans lequel ledit système polymère comprend moins de 0,5 % en poids de polymères ayant un poids moléculaire (Mw) allant de 399 000 à 800 000 g/mol.

19. Gomme de toffee selon l'une quelconque des revendications 1 à 18, dans lequel ledit système polymère est sensiblement exempt d'élastomères.

20. Gomme de toffee selon l'une quelconque des revendications 1 à 19, dans lequel ledit gomme de toffee est **caractérisé par** un module de stockage G' compris dans la plage allant de 100 Pa à 150 000 Pa tel que mesuré à une fréquence comprise dans la plage allant de 0,1 à 10 Hz.

21. Gomme de toffee selon l'une quelconque des revendications 1 à 20, dans lequel ledit gomme de toffee est **caractérisé par** un module de perte G" compris dans la plage allant de 1000 Pa à 200 000 Pa tel que mesuré à une fréquence comprise dans la plage allant de 0,1 à 10 Hz.

22. Gomme de toffee selon l'une quelconque des revendications 1 à 21, dans lequel ledit gomme de toffee est **caractérisé par** un rapport dudit module de perte G" sur ledit module de stockage G' compris dans la plage allant de 1 à 5.

23. Gomme de toffee selon l'une quelconque des revendications 1 à 22, dans lequel ledit module de stockage G' est inférieur audit module de perte G".

24. Gomme de toffee selon l'une quelconque des revendications 1 à 23, dans lequel ledit module de stockage G' est inférieur audit module de perte G" tel que mesuré à une fréquence de 1 Hz.

25. Gomme de toffee selon l'une quelconque des revendications 1 à 24, dans lequel ledit module de perte G" et ledit module de stockage G' sont tous deux inférieurs à 10 000 Pa tel que mesuré à une fréquence de 0,1 Hz.

26. Gomme de toffee selon l'une quelconque des revendications 1 à 25, dans lequel ledit module de perte G" est supérieur à 10 000 Pa tel que mesuré à une fréquence de 1 Hz.

27. Gomme de toffee selon l'une quelconque des revendications 1 à 26, dans lequel ledit module de stockage G' est inférieur à 40 000 Pa, de préférence inférieur à 30 000 Pa tel que mesuré à une fréquence de 1 Hz.

28. Gomme de toffee selon l'une quelconque des revendications 1 à 27, dans lequel ledit module de stockage G' diminue pendant au moins une partie d'un processus de mastication.

29. Gomme de toffee selon l'une quelconque des revendications 1 à 28, dans lequel ledit module de stockage G' diminue lors du processus de mastication pendant les 1 à 3 premières minutes.

30. Gomme de toffee selon l'une quelconque des revendications 1 à 29, dans lequel ledit module de perte G" diminue pendant au moins une partie du processus de mastication.

31. Gomme de toffee selon l'une quelconque des revendications 1 à 30, dans lequel ledit module de perte G" diminue lors du processus de mastication pendant les 1 à 3 premières minutes.

32. Gomme de toffee selon l'une quelconque des revendications 1 à 31, dans lequel ledit processus de mastication implique une fréquence de mastication à 1 Hz.

33. Gomme de toffee selon l'une quelconque des revendications 1 à 32, dans lequel ledit module de stockage G' et ledit module de perte G" sont mesurés à un couple d'oscillation allant de 8 à 12 µN•m.

34. Gomme de toffee selon l'une quelconque des revendications 1 à 33, dans lequel ledit gomme de toffee comprend une substance de gomme de toffee formée à partir d'un mélange dudit système polymère avec au moins un édulcorant et au moins un arôme.

35. Gomme de toffee selon l'une quelconque des revendications 1 à 34, dans lequel ledit gomme de toffee comprend du toffee ou du caramel en quantité comprise dans la plage allant de 1 % à 85 % en poids dudit gomme de toffee.

36. Gomme de toffee selon l'une quelconque des revendications 1 à 35, dans lequel au moins une partie dudit chocolat forme ou est compris dans au moins un module de chocolat dudit gomme de toffee.

37. Gomme de toffee selon l'une quelconque des revendications 1 à 36, dans lequel ledit au moins un module de chocolat comprend d'autres ingrédients.

38. Gomme de toffee selon l'une quelconque des revendications 1 à 37, dans lequel au moins l'un desdits au moins un module de chocolat forme un enrobage extérieur dudit gomme de toffee.

39. Gomme de toffee selon l'une quelconque des revendications 1 à 38, dans lequel ladite substance de gomme de toffee forme un noyau central et au moins l'un desdits au moins un module de chocolat est situé dans ledit noyau central.

40. Gomme de toffee selon l'une quelconque des revendications 1 à 39, dans lequel ledit noyau central comprend plusieurs desdits au moins un module de chocolat.

41. Gomme de toffee selon l'une quelconque des revendications 1 à 40, dans lequel ledit chocolat est appliqué en quantité comprise dans la plage allant de 5 % à 80 %, de préférence de 10 % à 60 % en poids dudit gomme de toffee.

42. Gomme de toffee selon l'une quelconque des revendications 1 à 41, dans lequel ledit chocolat contient du sucre.

43. Gomme de toffee selon l'une quelconque des revendications 1 à 42, dans lequel ledit chocolat est sensiblement sans sucre.

44. Gomme de toffee selon l'une quelconque des revendications 1 à 43, dans lequel ledit chocolat comprend du beurre de cacao en quantité allant de 15 % à 90 % en poids dudit chocolat.

45. Gomme de toffee selon l'une quelconque des revendications 1 à 44, dans lequel ledit chocolat est sélectionné parmi le groupe comprenant le chocolat noir, le chocolat au lait et le chocolat blanc, ou toute combinaison de ceux-ci.

46. Gomme de toffee selon l'une quelconque des revendications 1 à 45, dans lequel ledit chocolat est sélectionné parmi le groupe comprenant le chocolat édulcoré, le chocolat amer, le chocolat semi-amer et le chocolat sucré, ou toute combinaison de ceux-ci.

47. Gomme de toffee selon l'une quelconque des revendications 1 à 46, dans lequel ledit chocolat est du chocolat de couverture.

48. Gomme de toffee selon l'une quelconque des revendications 1 à 47, dans lequel ledit chocolat est un chocolat composé.

49. Gomme de toffee selon l'une quelconque des revendications 1 à 48, dans lequel au moins une partie dudit beurre de cacao dans ledit chocolat est remplacée par de la graisse ou de l'huile végétale.

50. Gomme de toffee selon l'une quelconque des revendications 1 à 49, dans lequel ledit chocolat est sensiblement dégraissé.

51. Gomme de toffee selon l'une quelconque des revendications 1 à 50, dans lequel ledit chocolat est sélectionné parmi le groupe comprenant le chocolat liquide, le chocolat solide et le chocolat en poudre, ou toute combinaison de ceux-ci.

52. Gomme de toffee selon l'une quelconque des revendications 1 à 51, dans lequel ledit module de chocolat formant un enrobage extérieur a été appliqué par un procédé d'enrobage sélectionné parmi le groupe comprenant l'enrobage par pulvérisation, l'enrobage en turbine, l'enrobage au rideau, ou toute combinaison de ceux-ci.

53. Gomme de toffee selon l'une quelconque des revendications 1 à 52, dans lequel ledit chocolat est substitué par un substitut du chocolat.

54. Gomme de toffee selon l'une quelconque des revendications 1 à 53, dans lequel ledit gomme de toffee comprend un pré-enrobage sous ledit enrobage extérieur.

55. Gomme de toffee selon l'une quelconque des revendications 1 à 54, dans lequel ledit enrobage extérieur fait l'objet d'un polissage.

56. Gomme de toffee selon l'une quelconque des revendications 1 à 55, dans lequel ledit gomme de toffee est combiné à d'autres ingrédients sélectionnés parmi le groupe comprenant la noix de coco, le riz, le maïs, le nougat, le massepain, le macaron, les amandes, les fruits, les baies, le caramel, la liqueur, les fruits à coque, les noisettes, les cacahuètes, les noix, la réglisse, la gomme au vin ou toute combinaison de ceux-ci.

57. Gomme de toffee selon l'une quelconque des revendications 1 à 56, dans lequel ledit gomme de toffee comprend au moins un ramollissant en quantité comprise dans la plage allant de 0,5 % à 25 %, de préférence dans la plage allant de 1 % à 18 %, et de manière préférée entre toutes dans la plage allant de 2 % à 12 % en poids dudit gomme de toffee.

58. Gomme de toffee selon l'une quelconque des revendications 1 à 57, dans lequel ledit au moins un ramollissant est sélectionné parmi le groupe comprenant la triacétine, la lécithine, les glycérides acétylés, les mono- et di-glycérides, ou toute combinaison de ceux-ci.

59. Gomme de toffee selon l'une quelconque des revendications 1 à 58, dans lequel ledit gomme de toffee comprend au moins un sirop d'un polyol en quantité de 1 % à 25 %, de préférence de 5 % à 20 % en poids dudit gomme de toffee.

60. Gomme de toffee selon l'une quelconque des revendications 1 à 59, dans lequel ledit système polymère comprend un/des plastifiant(s).

61. Gomme de toffee selon l'une quelconque des revendications 1 à 60, dans lequel le gomme de toffee comprend moins de 6 %, de préférence moins de 4 % en poids de charge, de préférence moins de 1 % en poids.

62. Gomme de toffee selon l'une quelconque des revendications 1 à 61, dans lequel le gomme de toffee est sensiblement exempt de charge.

63. Gomme de toffee selon l'une quelconque des revendications 1 à 62, dans lequel ledit gomme de toffee comprend ledit au moins un arôme en quantité de 0,001 % à 30 % en poids, et ledit au moins un édulcorant en quantité de 5 % à 80 % en poids.

64. Gomme de toffee selon l'une quelconque des revendications 1 à 63, dans lequel ledit arôme est un arôme de caramel comprenant une quantité comprise dans la plage allant de 1 % à 30 % en poids de ladite substance de gomme de toffee.

65. Gomme de toffee selon l'une quelconque des revendications 1 à 64, dans lequel ledit au moins un arôme est sensiblement à base d'huile et/ou sensiblement hydrophile.

66. Gomme de toffee selon l'une quelconque des revendications 1 à 65, dans lequel ledit au moins un édulcorant comprend du sucre.

67. Gomme de toffee selon l'une quelconque des revendications 1 à 66, dans lequel ledit sucre est sélectionné parmi le groupe comprenant les mono-, di-, ou polysaccharides et toute combinaison de ceux-ci.

68. Gomme de toffee selon l'une quelconque des revendications 1 à 67, dans lequel ledit sucre est sélectionné parmi le groupe comprenant le sucrose, le dextrose, le maltose, les dextrines, le tréhalose, le tagatose, le sucre inverti séché, le fructose, le lévulose, le galactose, les extraits secs de sirop de maïs, le polydextroseII, le polydextrose ultra, l'inuline, ou toute combinaison de ceux-ci.

69. Gomme de toffee selon l'une quelconque des revendications 1 à 68, dans lequel ledit au moins un édulcorant est un édulcorant artificiel.

70. Gomme de toffee selon l'une quelconque des revendications 1 à 69, dans lequel ledit édulcorant artificiel est sélectionné parmi le groupe comprenant le sorbitol, le mannitol, le maltitol, le xylitol, l'érythritol, le lactitol, l'isomalt, les dérivés de l'isomalt, ou toute combinaison de ceux-ci.

71. Gomme de toffee selon l'une quelconque des revendications 1 à 70, dans lequel ledit édulcorant artificiel est un édulcorant artificiel à haute intensité sélectionné parmi le groupe comprenant l'aspartame, les sels d'acésulfame, l'alitame, le néotame, le Twinsweet, la saccharine et ses sels, l'acide cyclamique et ses sels, la glycyrrhizine, les dihydrochalcones, la thaumatine, la monelline, le stévioside, le sucralose, ou toute combinaison de ceux-ci.

72. Gomme de toffee selon l'une quelconque des revendications 1 à 71, dans lequel ledit gomme de toffee comprend en outre des ingrédients actifs.

73. Gomme de toffee selon l'une quelconque des revendications 1 à 72, dans lequel ledit gomme de toffee forme une pastille comprenant un système polymère à mâcher.

74. Gomme de toffee selon l'une quelconque des revendications 1 à 73, dans lequel ledit gomme de toffee est formé dans des formes telles que des noyaux, ellipses, boules, cylindres, carrés, rectangles, hexagones, bandes, paraboles, beignets, anneaux, oursons et/ou multi-modules.

75. Gomme de toffee selon l'une quelconque des revendications 1 à 74, dans lequel ledit gomme de toffee pèse de ¼ gramme à 10 grammes.

76. Gomme de toffee selon l'une quelconque des revendications 1 à 75, dans lequel ledit gomme de toffee pèse de ½ gramme à 5 grammes.

77. Gomme de toffee selon l'une quelconque des revendications 1 à 76, dans lequel ledit gomme de toffee est muni d'un enrobage extérieur.

78. Gomme de toffee selon l'une quelconque des revendications 1 à 77, dans lequel ledit enrobage est sélectionné parmi le groupe comprenant un enrobage dur, un enrobage tendre et un enrobage en film comestible.

79. Gomme de toffee selon l'une quelconque des revendications 1 à 78, dans lequel ledit gomme de toffee est fourré.

80. Gomme de toffee selon l'une quelconque des revendications 1 à 79, dans lequel ledit gomme de toffee est comprimé.

81. Procédé de fabrication d'un gomme de toffee selon l'une quelconque des revendications 1 à 80, de sorte que le produit est fabriqué par un traitement par lots ou un procédé d'extrusion.

82. Procédé de fabrication d'un gomme de toffee selon la revendication 81 avec un enrobage de chocolat, ledit procédé comprenant les étapes consistant à appliquer du chocolat sous forme liquide à la surface d'un noyau central de gomme de toffee et à soumettre le gomme de toffee enrobé de chocolat à une température qui cause le durcissement du chocolat liquide.
